# EUROPEAN PATENT APPLICATION

(11) **EP 2 460 788 A1**
(43) Date of publication of application: **06.06.2012**
(21) Application number: 10804541.0
(22) Date of filing: 30.07.2010
(51) Int. Cl.: C07C 251/24, C09K 11/06, H01L 51/50, C07F 15/00

(54) **METAL COMPLEX, COMPOSITION COMPRISING SAME AND LIGHT-EMITTING ELEMENT USING SAME**

(30) Priority: 31.07.2009 JP 2009180144
(71) Applicant: Sumitomo Chemical Company, Limited, Tokyo 104-8260 (JP); Tokyo Institute of Technology, Tokyo 152-8550 (JP)
(72) Inventor: IKEDA Tomiki, Yokohama-shi Kanagawa 226-8503 (JP); KINOSHITA Motoi, Yokohama-shi Kanagawa 226-8503 (JP); NAM Yunmi, Yokohama-shi Kanagawa 226-8503 (JP)
(74) Representative: Duckworth, Timothy John
(86) International application number: PCT/JP2010/062893
(87) International publication number: WO 2011/013795

(57) **Abstract**

The invention provides a metal complex having a structure represented by the following formula (1): 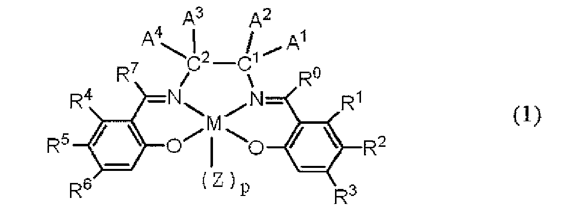 wherein M represents a metal atom such as copper; C¹ and C² are each an sp3 carbon atom; A¹-A⁴ each independently represent hydrogen, etc; this is with the proviso that A¹ and A³ may bond together to form a C4 or greater alkylene group and form a ring together with C¹ and C²; the alkylene group may be optionally substituted; R⁰-R⁷ each independently represent hydrogen, etc; Z represents a monovalent monodentate ligand, and p is the number of monodentate ligands, represented by ("valency of central metal atom M" - 2).

## Description

### Technical Field

The present invention relates to a metal complex, to a composition comprising the metal complex and a charge transporting organic compound, and to a light emitting element comprising the metal complex.

### Background Art

A large number of fluorescent materials and phosphorescent materials have been proposed as luminescent organic materials useful for fabrication of organic electroluminescence (EL) elements. Phosphorescent materials produce luminescence with higher efficiency than fluorescent materials, and they are therefore being actively researched in recent years and many luminescent metal complexes have been developed. For example, orthometalated complexes comprising iridium as the central metal (Ir(ppy)₃) have been proposed as metal complexes exhibiting green luminescence (Non-patent document 1). Also, [[2,2'[1,2-phenylenebis](nitrilomethylidyne)]bis[phenolate]]-NN',O,O'] platinum(II) having platinum as the central metal has been reported as a metal complex exhibiting red luminescence (Non-patent document 2).

Technical development has also been carried out in the prior art to obtain polarized luminescence, as a new function for organic EL elements. For example, polarized EL elements have been proposed which employ a nematic liquid crystal compound with an acrylate polymerizable group and a fluorescent dye as a luminescent material (Patent document 1).

### Citation List

### Patent Literature

[Patent document 1] Japanese Patent Public Inspection HEI No. 10-508979

### Non Patent Literature

[Non-patent document 1] APPLIED PHYSICS LETTERS, Vo1.75, p.4 (1999)
[Non-patent Document 2] M. E. Ivanova et al., Zhur. Fiz. Khim., Vo1.65, 1991, p.2957-2964

### Summary of Invention

### Technical Problem

However, in most cases of metal complexes that have been developed to date, the compositions comprising them are suited for organic EL elements in which the luminescent layer is formed by vapor deposition, while few are suited for organic EL elements in which the luminescent layer is formed using a wet process such as an ink-jet system or spin coating. A demand therefore exists for a metal complex that produces high efficiency luminescence, and is suitable for formation of a luminescent layer by a wet process. The conventionally known metal complexes, however, do not adequately meet this demand in terms of luminescent performance.

It is therefore an object of the invention to provide a metal complex that permits application of a wet process and that exhibits sufficient luminescent performance, as well as a composition comprising it.

### Solution to Problem

The invention provides, firstly, a metal complex having a structure represented by the following formula (1):

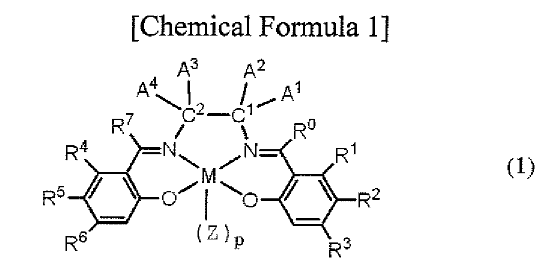

wherein M is a metal atom selected from the group consisting of copper, zinc, ruthenium, silver, osmium, rhenium, iridium, platinum, gold and lanthanum; C¹ and C² are each an sp3 carbon atom; A¹-A⁴ each independently represent hydrogen or a C3 or greater alkyl group, with at least 2 of them representing C3 or greater alkyl groups; this is with the proviso that A¹ and A³ may bond together to form a C4 or greater alkylene group and form a ring together with C¹ and C²; the alkylene group may be optionally substituted; R⁰-R⁷ each independently represent hydrogen, a halogen atom, a C6 or greater alkyl group optionally substituted with fluorine, a C12 or greater aralkyl group optionally substituted with fluorine, a C12 or greater alkaryl group optionally substituted with fluorine, a C6 or greater alkoxy group optionally substituted with fluorine, a C12 or greater arylalkoxy group optionally substituted with fluorine or a C12 or greater alkoxyaryl group optionally substituted with fluorine, and at least one of R⁰-R⁷ is a C6 or greater alkyl group optionally substituted with fluorine, a C12 or greater aralkyl group optionally substituted with fluorine, a C12 or greater alkaryl group optionally substituted with fluorine, a C6 or greater alkoxy group optionally substituted with fluorine, a C12 or greater arylalkoxy group optionally substituted with fluorine or a C12 or greater alkoxyaryl group optionally substituted with fluorine; Z represents a monovalent monodentate ligand, and p is the number of monodentate ligands, represented by ("valency of central metal atom M" - 2).

The invention provides, secondly, a composition comprising the metal complex and a charge transporting organic compound.
The invention provides, thirdly, a film obtained using the aforementioned metal complex or composition.
The invention provides, fourthly, a light emitting element comprising the film.
The invention provides, fifthly, a planar light source employing the light emitting element.
The invention provides, sixthly, a lighting fixture employing the light emitting element.

### Advantageous Effects of Invention

The metal complex of the invention and a composition comprising it can yield a luminescent layer exhibiting sufficient luminous efficiency by a wet process, in the fabrication of an organic electroluminescence element or the like.
Furthermore, according to a preferred embodiment, the metal complex of the invention and a composition comprising it allow polarized EL luminescence to be obtained by orientation treatment in the element fabrication process.

### Description of Embodiments

The present invention will now be explained in detail. In the explanation which follows, "number-average molecular weight" and "weight-average molecular weight" refer to the number-average molecular weight and weight-average molecular weight in terms of polystyrene, measured by size-exclusion chromatography (SEC).

### <Metal complex>

The metal complex of the invention is represented by the above formula (1).

The central metal atom M in the metal complex of the invention is a metal atom selected from the group consisting of copper, zinc, ruthenium, silver, osmium, rhenium, iridium, platinum, gold and lanthanum, but from the viewpoint of high luminous efficiency, it is preferably a metal atom selected from the group consisting of ruthenium, silver, osmium, rhenium, iridium, platinum, gold and lanthanum, and more preferably a metal atom selected from the group consisting of iridium and platinum.

In the above formula (1), C¹ and C² are each an sp3 carbon atom. A¹-A⁴ each independently represent hydrogen or a C3 or greater alkyl group, with at least 2 of them representing C3 or greater alkyl groups. This is with the proviso that A¹ and A³ may bond together to form a C4 or greater alkylene group and form a ring together with C¹ and C². The alkylene group may be optionally substituted. R⁰-R⁷ each independently represent hydrogen, a halogen atom, a C6 or greater alkyl group optionally substituted with fluorine, a C 12 or greater aralkyl group optionally substituted with fluorine, a C12 or greater alkaryl group optionally substituted with fluorine, a C6 or greater alkoxy group optionally substituted with fluorine, a C12 or greater arylalkoxy group optionally substituted with fluorine or a C12 or greater alkoxyaryl group optionally substituted with fluorine, and at least one of R⁰-R⁷ is a C6 or greater alkyl group optionally substituted with fluorine, a C12 or greater aralkyl group optionally substituted with fluorine, a C12 or greater alkaryl group optionally substituted with fluorine, a C6 or greater alkoxy group optionally substituted with fluorine, a C12 or greater arylalkoxy group optionally substituted with fluorine or a C12 or greater alkoxyaryl group optionally substituted with fluorine.

Z represents a monovalent monodentate ligand, and n is the number of monodentate ligands, represented by ("valency of central metal atom M" - 2).

### - Explanation of A¹ to A⁴ -

A¹-A⁴ each independently represent hydrogen or a C3 or greater alkyl group, with at least 2 of them representing C3 or greater alkyl groups. Specific examples of alkyl groups represented by A¹-A⁴ include C3-15 alkyl groups such as propyl, butyl, pentyl, hexyl, heptyl, octyl, nonyl, decyl, undecyl and dodecyl.
A¹ and A³ may bond together to form a C4 or greater alkylene group (preferably a tetramethylene group) and form a ring together with C¹ and C², and when a ring is formed, a heteroatom may be included in the ring portion. When A¹ and A³ form a ring, it may be a saturated six-membered ring or saturated heterocyclic ring, and the following structure is preferred as the ring portion.

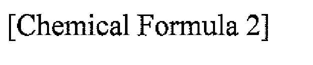

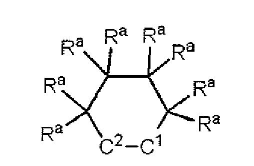

In the formula, R^{a} represents hydrogen, a C1 or greater alkyl group optionally substituted with fluorine, a C6 or greater aryl group, a C7 or greater aralkyl group optionally substituted with fluorine, a C7 or greater alkaryl group optionally substituted with fluorine, a C1 or greater alkoxy group optionally substituted with fluorine, a C7 or greater arylalkoxy group optionally substituted with fluorine or a C7 or greater alkoxyaryl group optionally substituted with fluorine. Multiple R^{a} groups may be the same or different. In the case of a saturated six-membered ring, C¹ and C² are preferably chiral carbon atoms. Also, at least one R^{a} is preferably a C3 or greater alkyl group optionally substituted with fluorine, a C7 or greater aralkyl group optionally substituted with fluorine, a C7 or greater alkoxyaryl group optionally substituted with fluorine, a C3 or greater alkoxy group optionally substituted with fluorine, a C7 or greater arylalkoxy group optionally substituted with fluorine or a C7 or greater alkoxyaryl group optionally substituted with fluorine.

The alkyl group represented by R^{a} may be straight-chain or branched, but is preferably straight-chain. Specific examples of alkyl groups include C1-15 alkyl groups such as methyl, ethyl, propyl, butyl, pentyl, hexyl, heptyl, octyl, nonyl, decyl, undecyl and dodecyl, with C8-12 alkyl groups being preferred.
Aralkyl and alkaryl groups represented by R^{a} preferably have phenyl or phenylene groups, examples of which include groups represented by the following formulas. 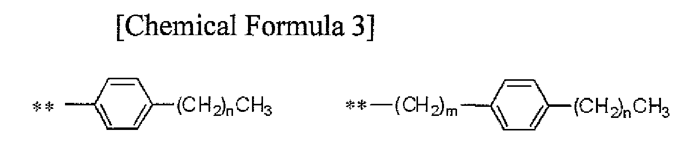 (In the formulas, m and n each independently represent an integer of 0-15 and preferably an integer of 0-12, and "**" represents a bond with a ring.)

The alkoxy group represented by R^{a} may be straight-chain or branched, but is preferably straight-chain. Specific examples of alkoxy groups include C1-15 alkoxy groups such as methoxy, ethoxy, propyloxy, butyloxy, pentyloxy, hexyloxy, heptyloxy, octyloxy, nonyloxy, decyloxy and undecyloxy, with C6-12 alkoxy groups being preferred.
Arylalkoxy and alkoxyaryl groups represented by R^{a} preferably have phenyl or phenylene groups, and 6-12 carbon atoms in the alkoxy group portion, examples of which include groups represented by the following formulas. 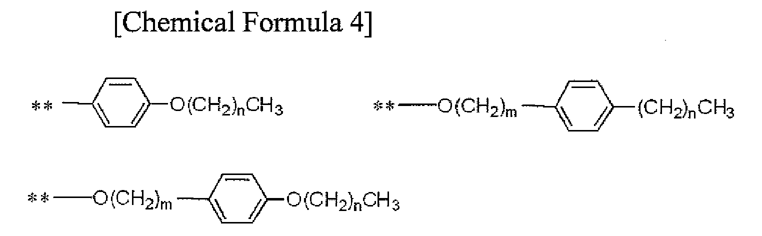 (In the formulas, m, n and ** have the same meanings as above.)

### - Explanation of substituents R⁰ to R⁷ -

The following explanation assumes that R⁰-R⁷ are substituents other than hydrogen, and that they are not substituted with fluorine.
The alkyl groups represented by R⁰-R⁷ may be straight-chain or branched, but preferably the number of carbon atoms of the longest alkyl group among R⁰-R⁷ (that is, the largest number of carbon atoms in the straight-chain) is 10 or greater. The number of carbon atoms of the alkyl groups will usually be 6-20, and is preferably 6-15. Alkyl groups include hexyl, heptyl, octyl, nonyl, decyl, undecyl, dodecyl, tridecyl, tetradecyl and pentadecyl, and among these, hexyl, heptyl, octyl, nonyl, decyl and undecyl.

The aralkyl and alkaryl groups represented by R⁰-R⁷ may be straight-chain or branched, but preferably the alkyl group portions are straight-chain. Aralkyl and alkaryl groups preferably have phenyl or phenylene groups, examples of which include groups represented by the following formulas. 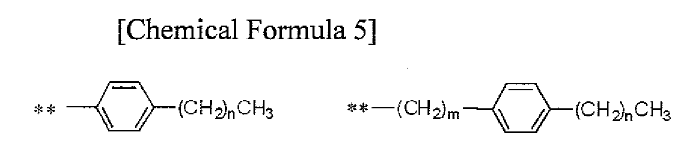 (In the formulas, m, n and ** have the same meanings as above.)

The alkoxy groups represented by R⁰-R⁷ may be straight-chain or branched, but are preferably straight-chain. The number of carbon atoms of the alkoxy group will usually be 6-15 and preferably 8-12. Specific examples of alkoxy groups include alkoxy groups with up to 15 carbon atoms, such as hexyloxy, heptyloxy, octyloxy, nonyloxy, decyloxy and lauryloxy, with hexyloxy, octyloxy, decyloxy and undecyloxy groups being preferred.

The arylalkoxy and alkoxyaryl groups represented by R⁰-R⁷ may be straight-chain or branched, but preferably the alkoxy group portions are straight-chain. Also, the arylalkoxy and alkoxyaryl groups preferably have phenyl or phenylene groups, and the numbers of carbon atoms of the alkoxy group portions are preferably 8-12. Examples of arylalkoxy and alkoxyaryl groups include groups represented by the following formulas. 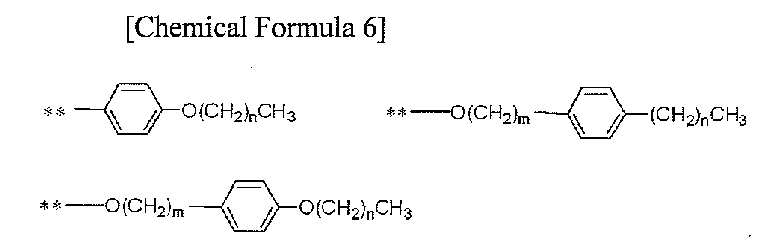 (In the formulas, m, n and ** have the same meanings as above.)
When R⁰-R⁷ are substituents other than hydrogen, the substituents are preferably alkyl or alkoxy groups.

### - Explanation of optional ligand Z -

When the valency of the central metal M in the metal complex of the invention is greater than 2, a monovalent monodentate ligand Z may be further coordinated with the central metal M. The monovalent monodentate ligand preferably has an aromatic ring (is a monodentate ligand with an aromatic ring), and more preferably the aromatic ring structure (aromatic ring) includes the ligand atom, and more preferably, the ligand atom in the aromatic ring is a carbon atom or nitrogen atom and the aromatic ring is a fused ring. The number p of the monodentate ligand Z is equal to ("valency of central metal atom M" - 2). The valency of the metal atom M will differ depending on the type of metal, and for example, rhenium can have a number of different valencies (+2 to +7), the metal valency of 2 is preferred from the viewpoint of planarity of the complex structure, in order to exhibit orientation.

### · Examples of monodentate ligands:

The following (S-1) are examples of monovalent monodentate ligands. 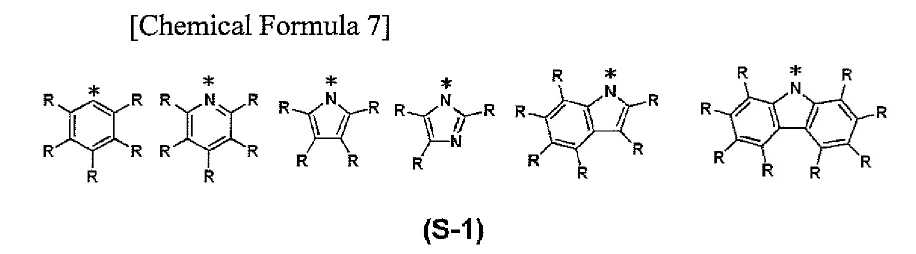 [In formula (S-1), "*" represents an atom coordinated with a metal, and multiple R groups each independently represent hydrogen, C1-8 alkyl, C1-8 alkoxy, C6-10 aryl, C7-13 aryloxy, C7-13 aralkyl, C7-13 alkaryl, C7-13 arylalkoxy, C7-13 alkoxyaryl or a halogen atom.]

The alkyl, alkoxy, aralkyl, alkaryl, arylalkoxy and alkoxyaryl groups mentioned above are preferably the same as mentioned for R⁰-R⁷, and phenyl and phenyloxy groups are preferred as aryl and aryloxy groups.

Of the metal complexes having the structure represented by the above formula (1), there are preferred metal complexes with the structure represented by the following formula (2). 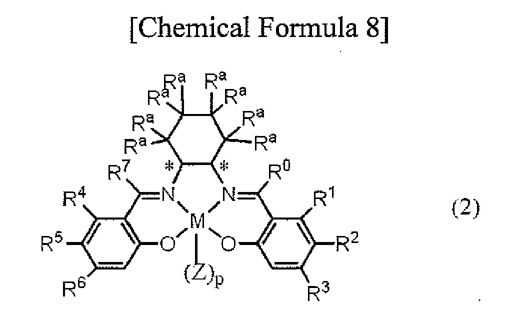 [In the formula, R^{a} has the same meaning as above. R⁰-R⁷, Z and p have the same definitions as in the formula (1), and "*" represents a chiral carbon atom.]

More preferred is a metal complex having a structure represented by the following formula (3). 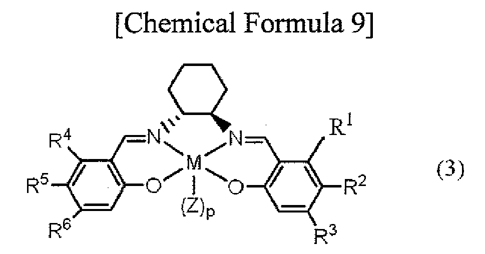 [In the formula, M is a platinum atom or iridium atom, p being 0 when M is a platinum atom and p being 1 when M is an iridium atom, and at least one of R¹-R³ is a C8-15 alkoxy group while at least one of R⁴-R⁶ is a C8-15 alkoxy group.]

### <Examples of metal complexes>

The following metal complexes may be mentioned as metal complexes.

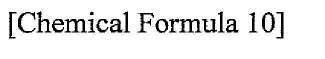

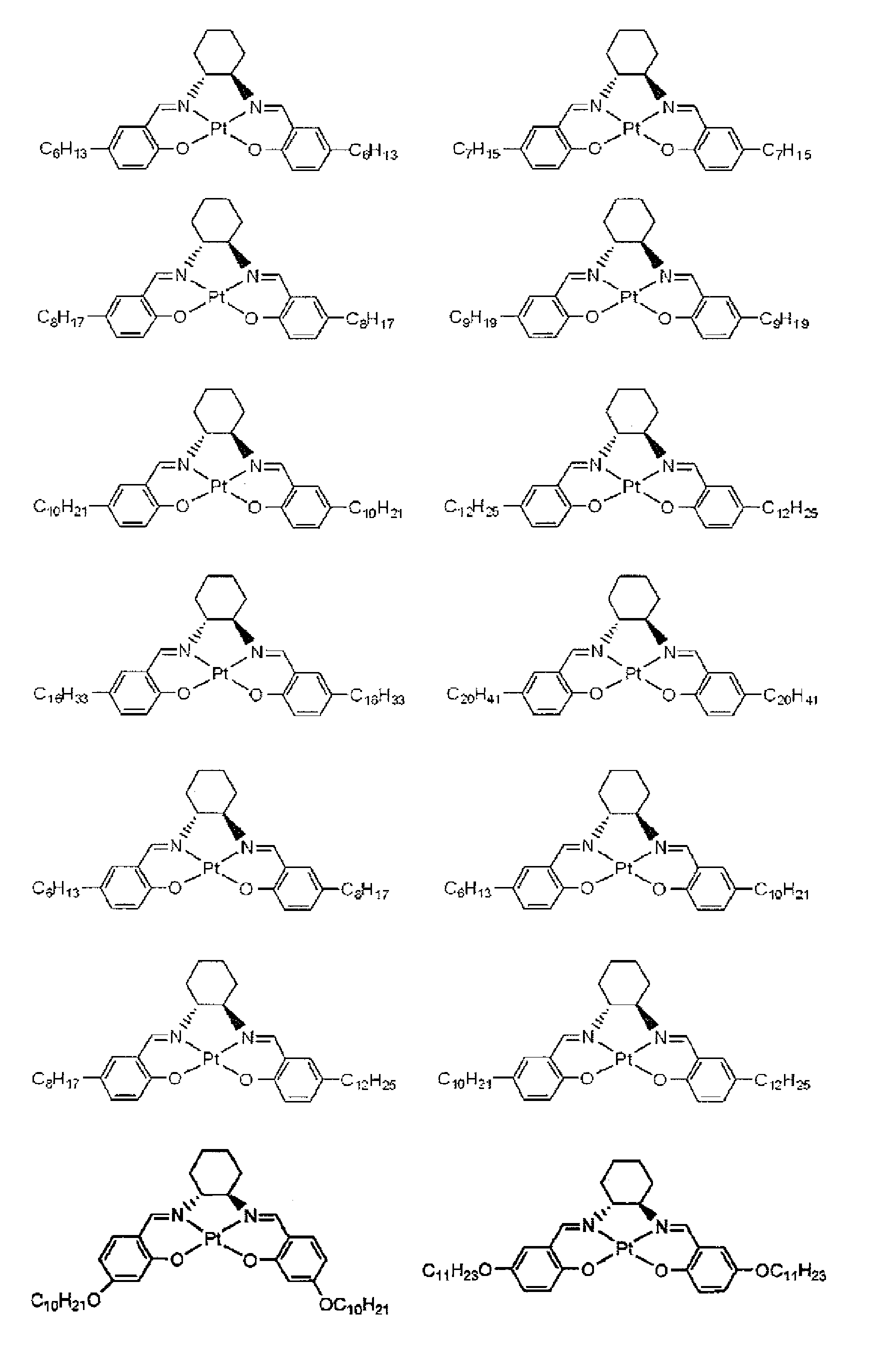

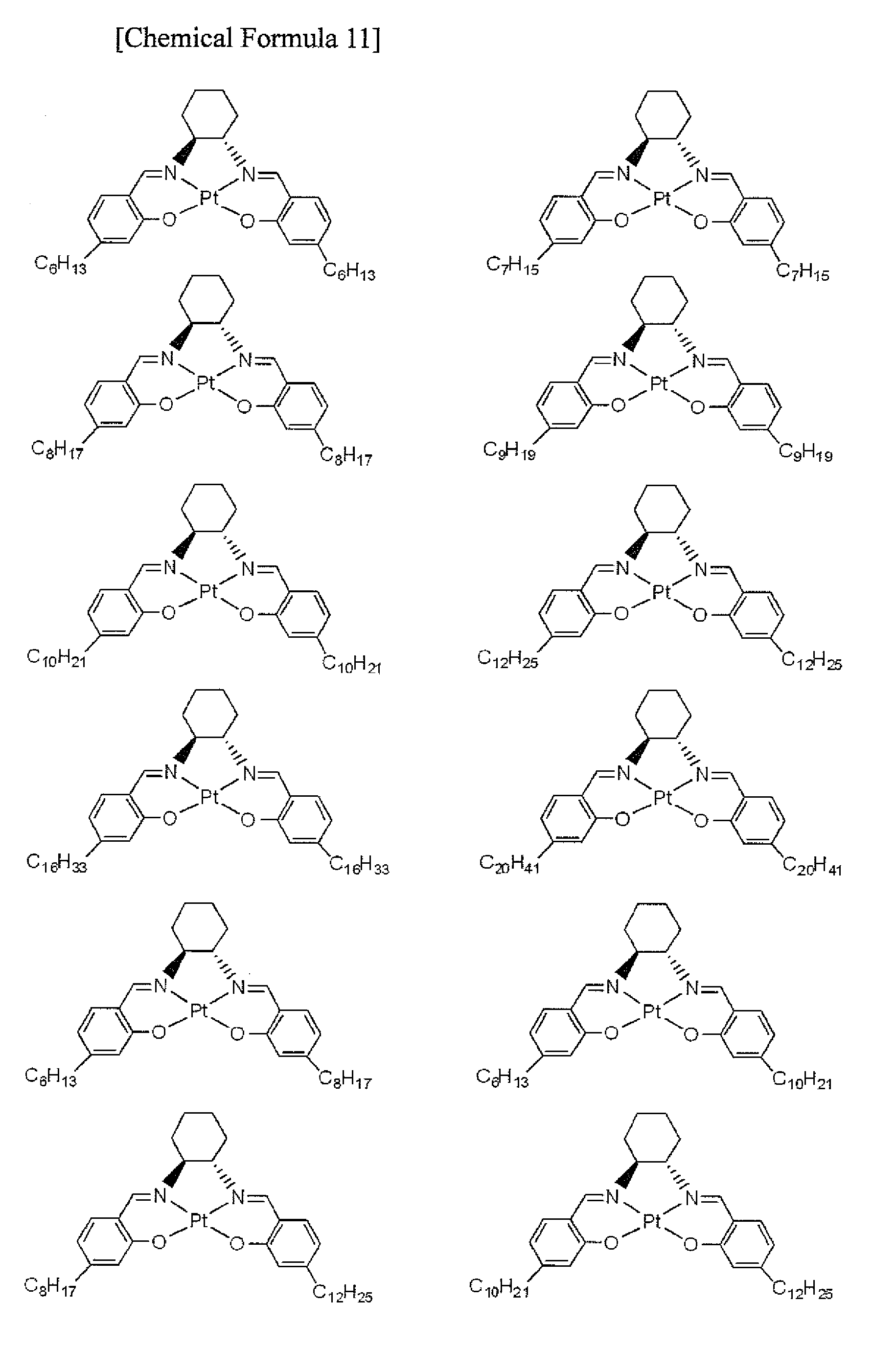

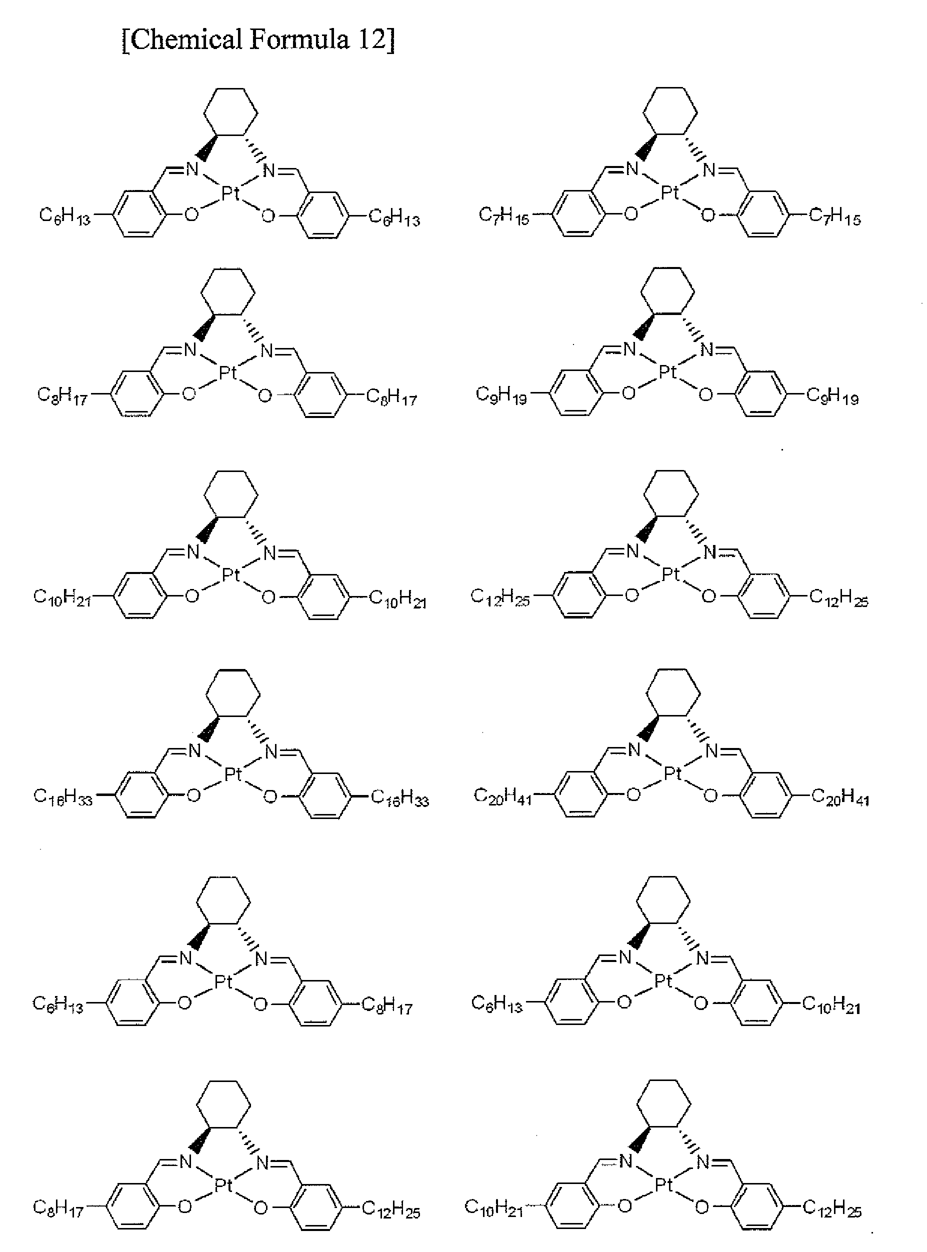

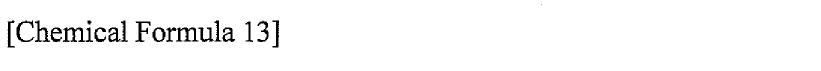

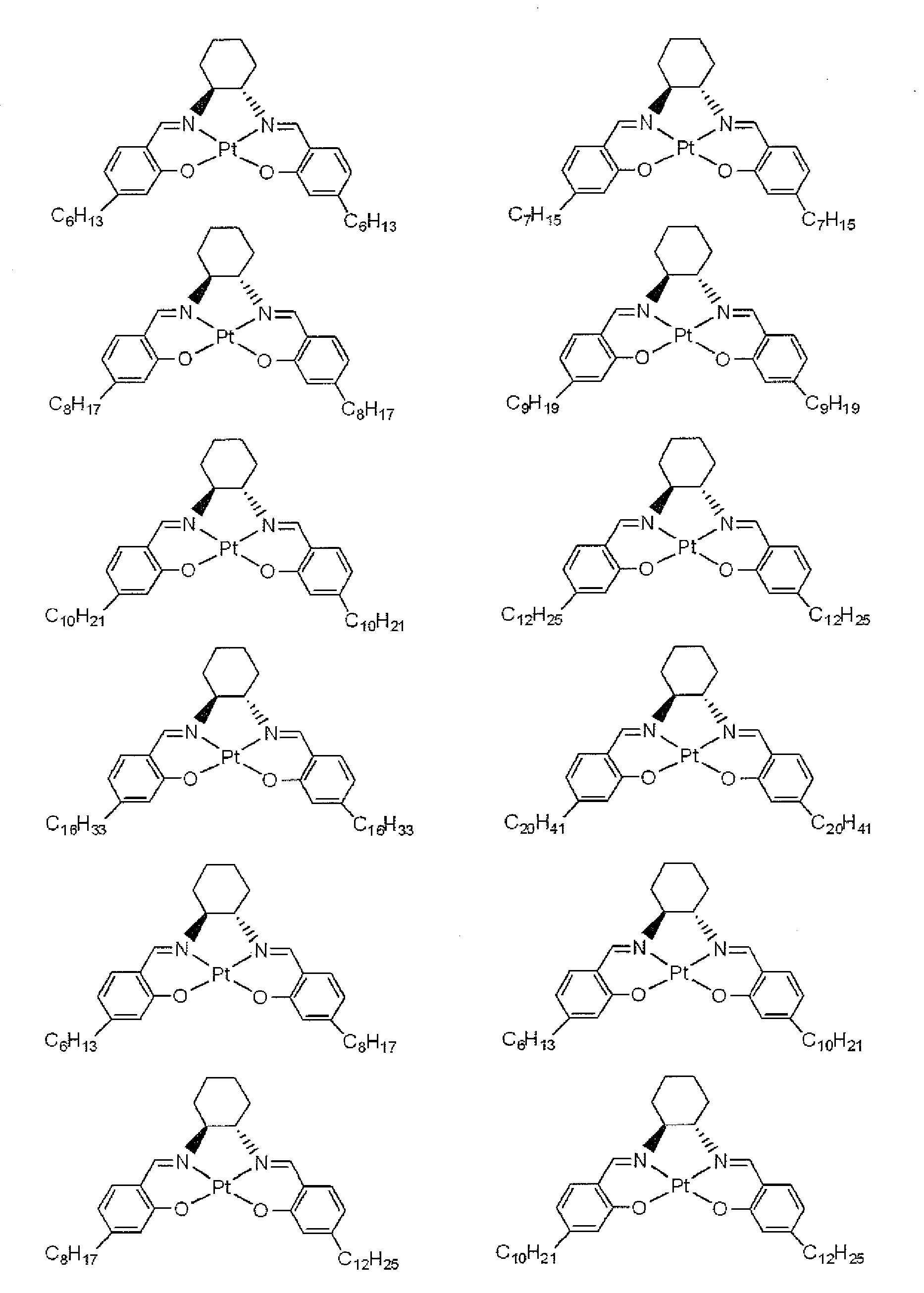

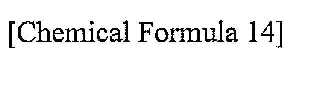

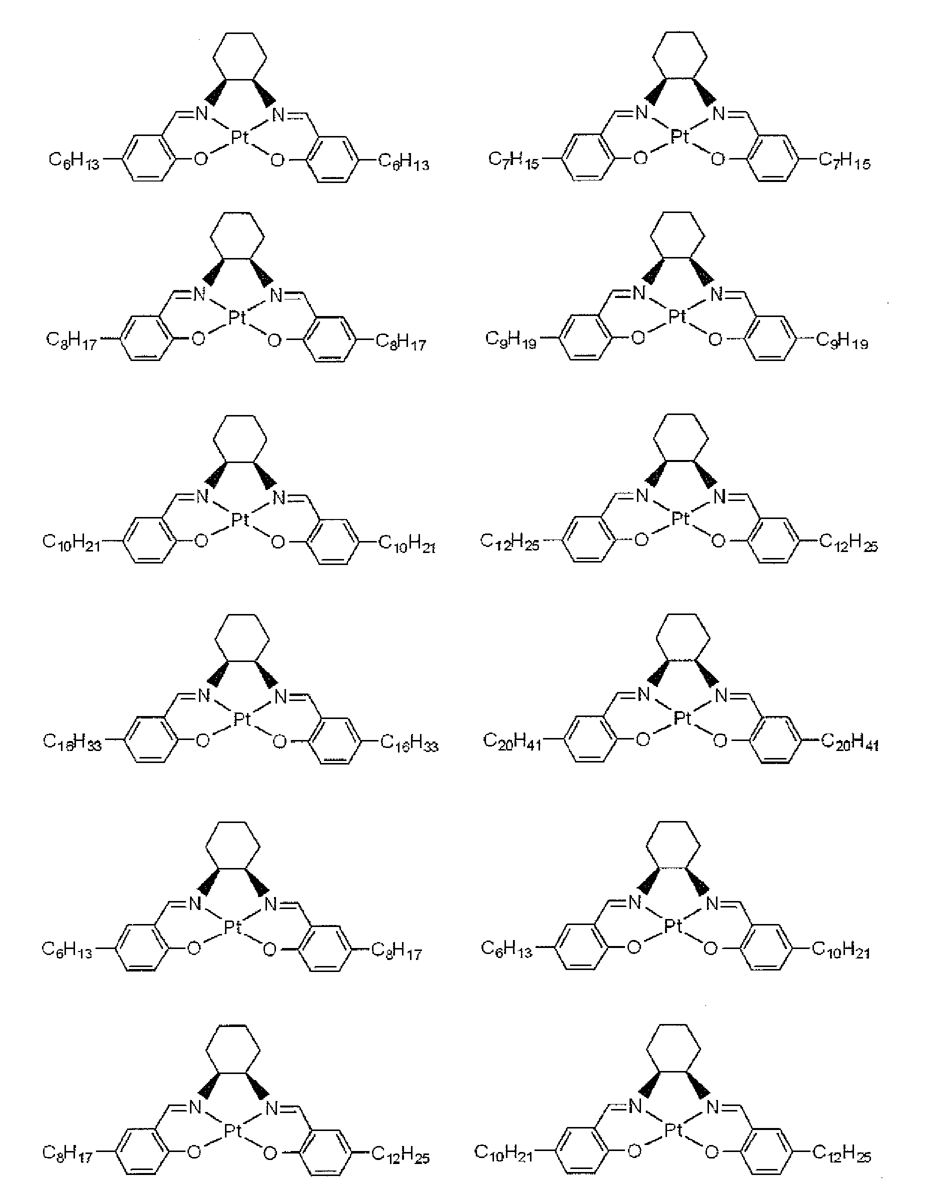

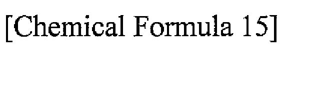

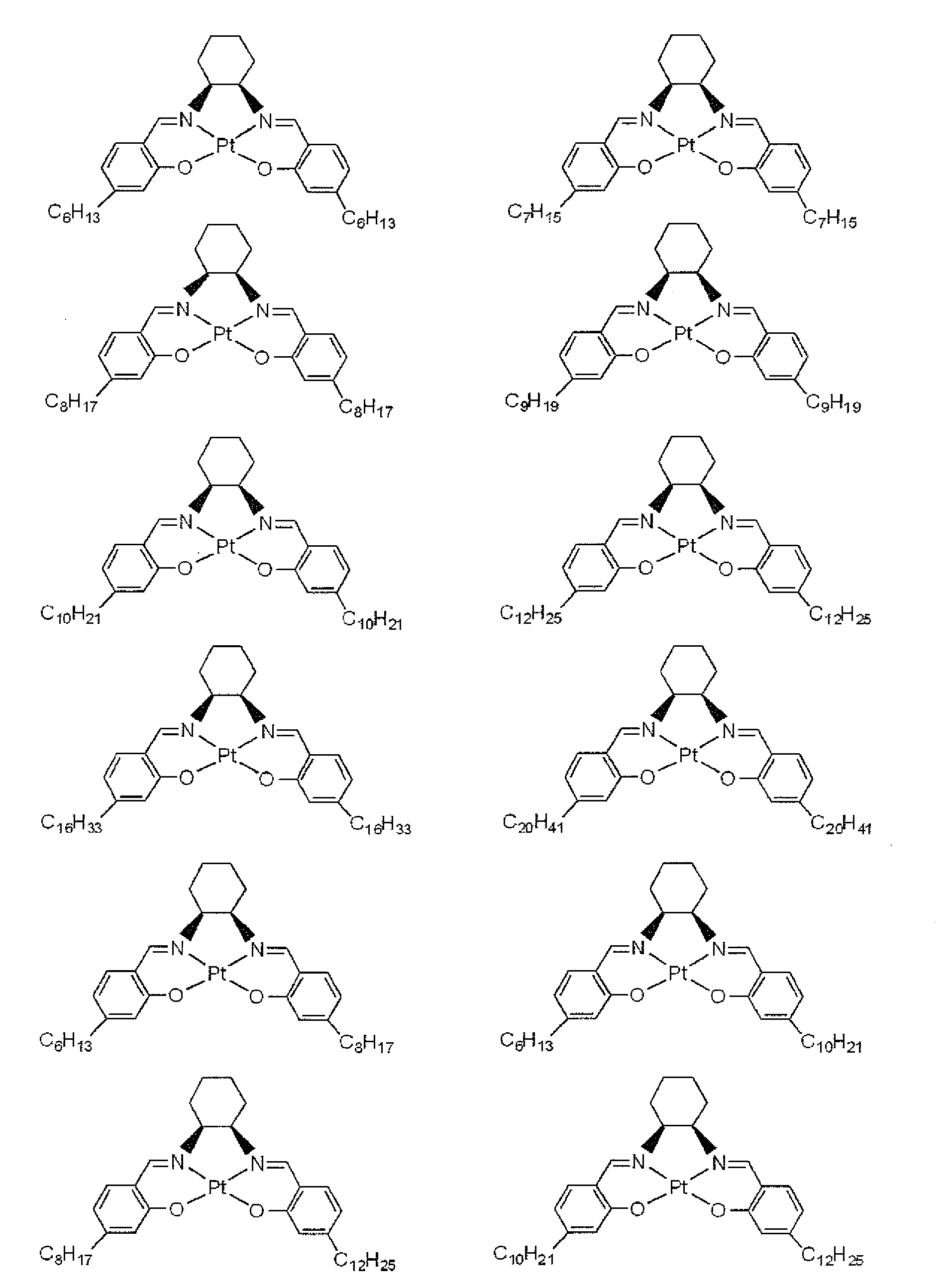

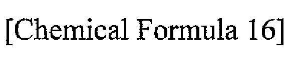

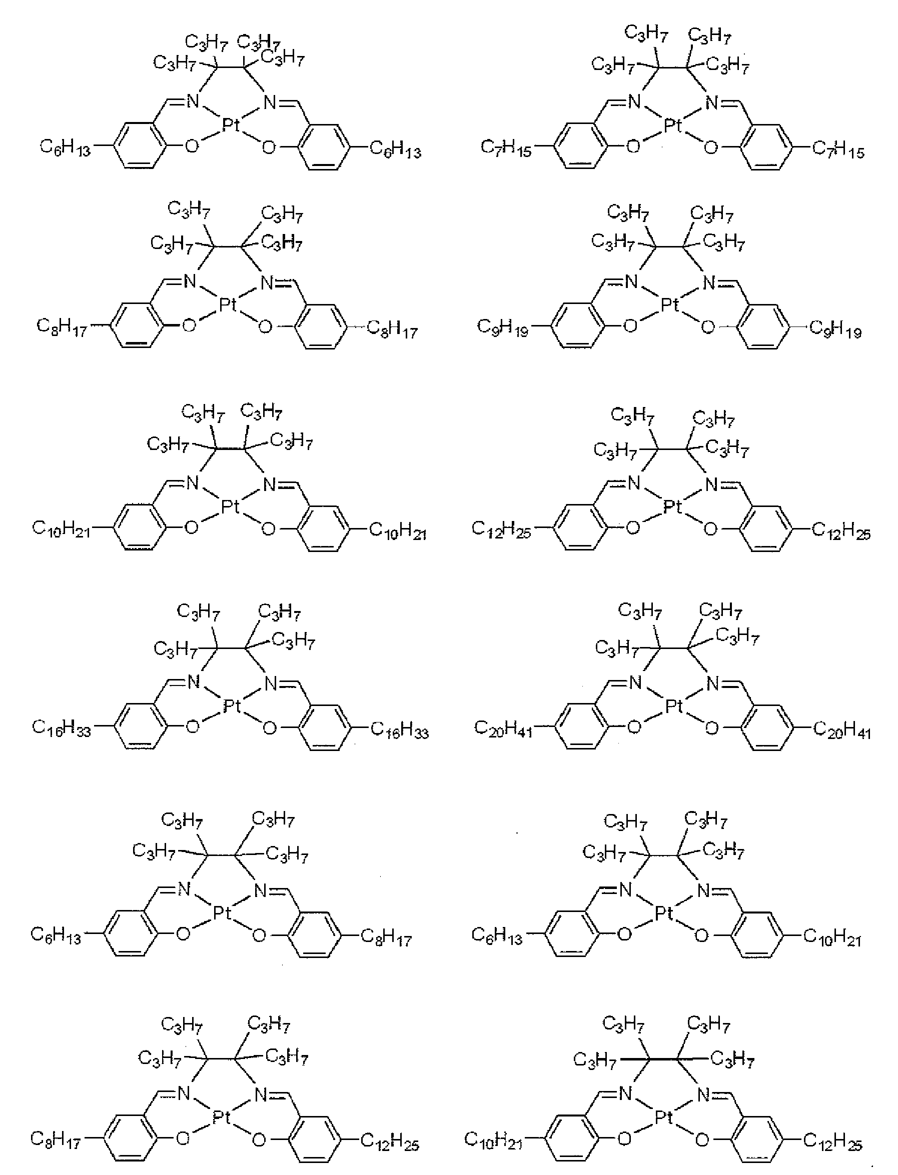

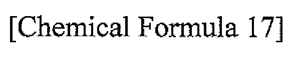

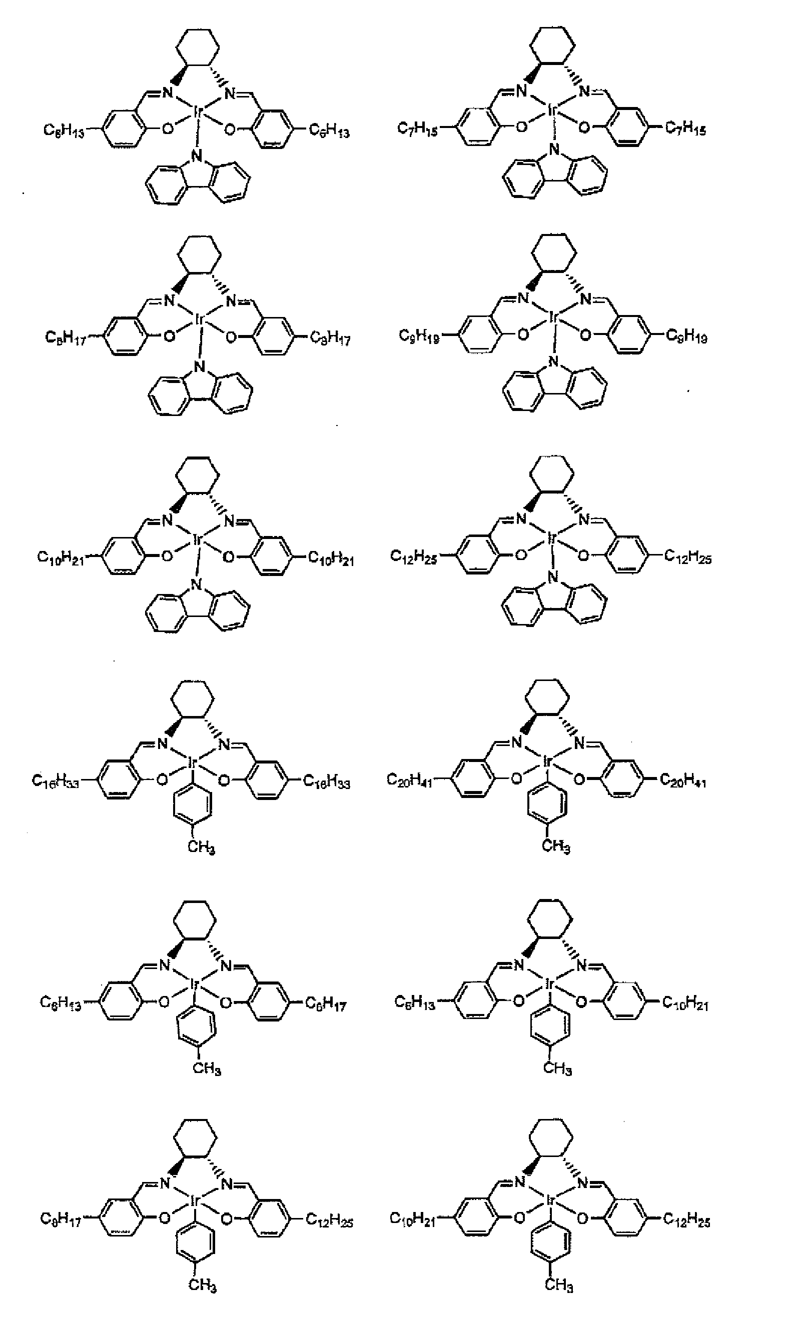

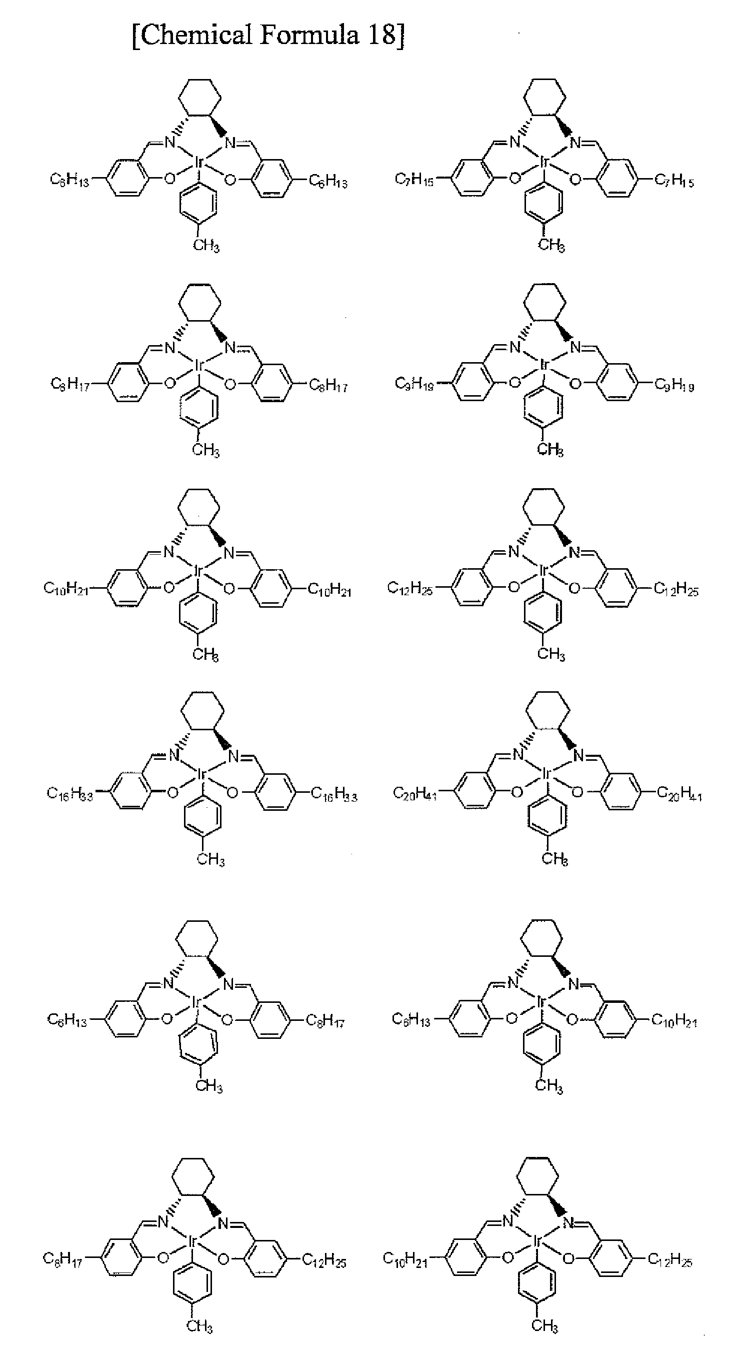

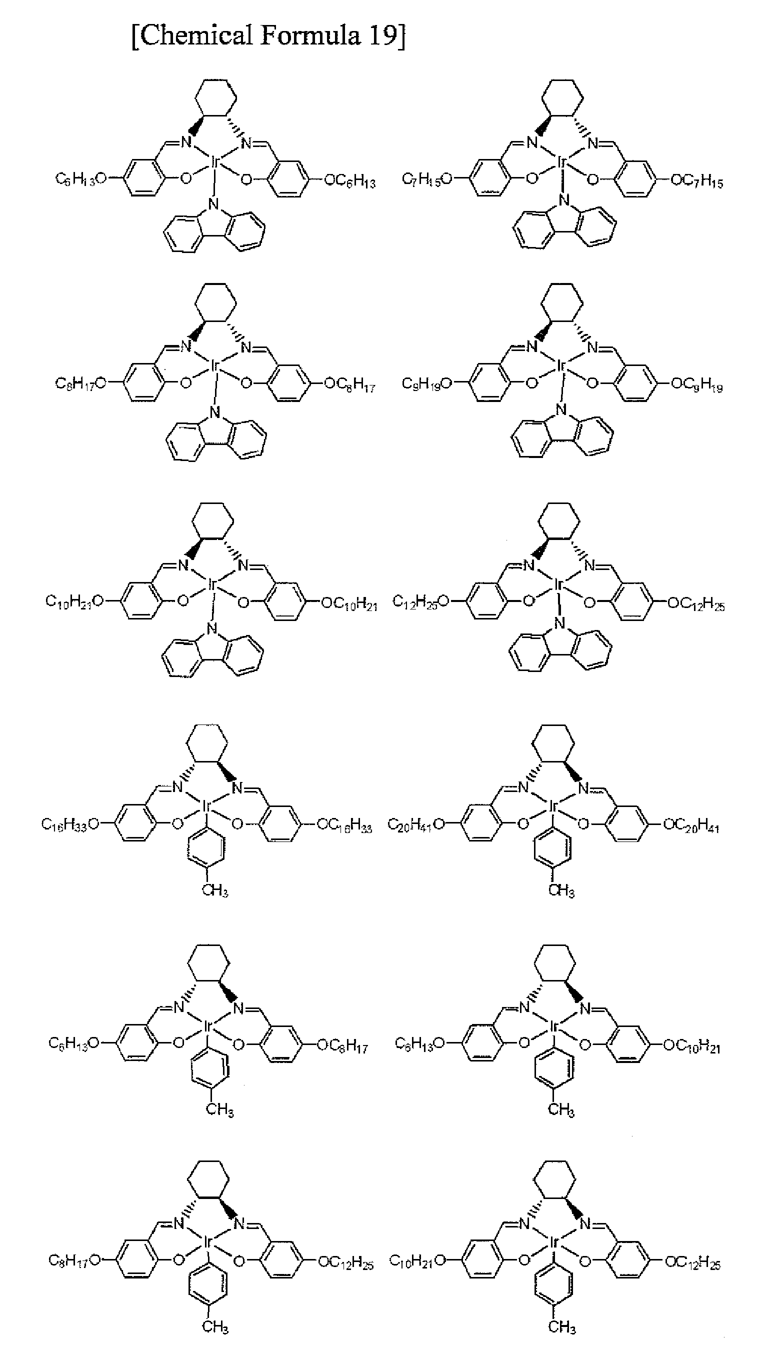

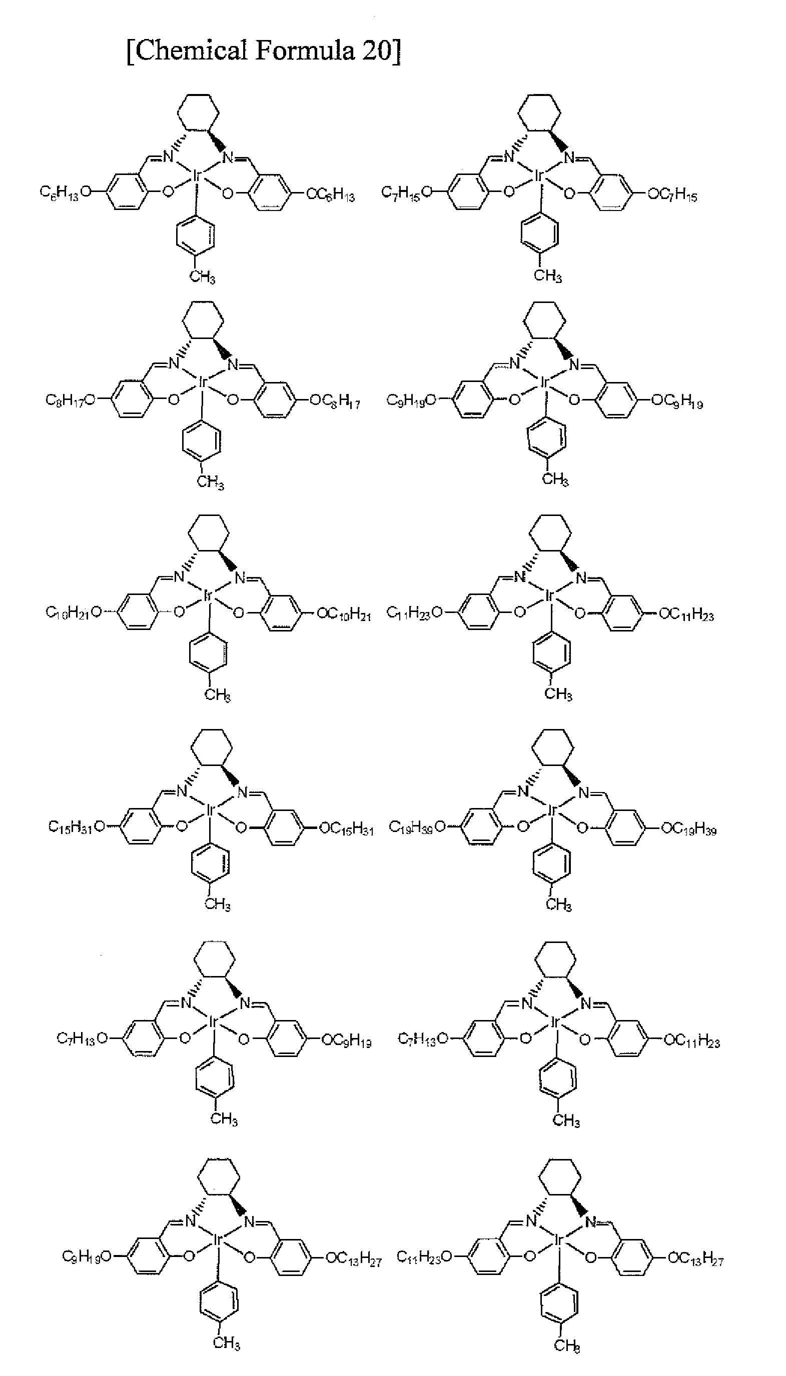

From the viewpoint of stable high efficiency luminescence, these metal complexes are preferably neutral metal complexes with short lifetimes of the triplet excited state, which tend to avoid forbidden transitions.

The metal complex may also have orientation. From the viewpoint of increasing the orientation, preferably at least two of R⁰-R⁷ include a group with a long carbon chain (preferably C6-12 alkyl or C6-12 alkoxy groups). In this case, R² and R⁵, or R³ and R⁶, are preferably groups with long carbon chains. Also, when R⁰-R⁷ do not have 2 or more groups (only 0 or 1) with long carbon chains, the orientation can be increased if at least two of A¹-A⁴ are groups with long carbon chains or groups containing aromatic rings.

A process for production of such a metal complex will now be described.
The metal complex may be synthesized by reacting the ligand compound with the metal compound in a solution.

The complexing method (that is, the method of reacting the ligand compound with the metal compound in the solution) may be, for example, the method described in Inorg. Chem. 2006, 45, 10976, Dalton Trans. 2004, 2237.
The complexing reaction may usually be from -80°C to 300°C, and preferably the reaction is conducted between the melting point of the solvent and 200°C. When the reaction is conducted at above the boiling point of the solvent, a pressurized reaction may be used that can withstand the vapor pressure of the solvent at the reaction temperature. The heating method may involve ordinary heating with an oil bath, heater or the like, or heating using a microwave. The reaction time may be the time required for the separate reactions, and it will usually be 30 minutes to 48 hours, and preferably 12 hours to 24 hours.

The ligand compound may be synthesized by the method described in Inorg. Chem. 2006, 45, 10976, Dalton Trans. 2004, 2237, for example.
The metal compound used may be either an inorganic metal compound or organometallic compound. Examples of inorganic metal compounds include metal halides such as metal chlorides, metal bromides and metal iodides, and metal acid halides such as sodium metal acid chloride and potassium metal acid chloride. When the metal is platinum, inorganic metal compounds include platinum chloride, platinum bromide, platinum iodide, sodium chloride platinate, potassium chloride platinate and potassium bromide platinate. When the metal is platinum, organometallic compounds include dichloro(1,5-cyclooctadiene)platinum(II), bis(benzonitrile)dichloroplatinum(II) and dichlorobis(dimethyl sulfoxide)platinum(II). These metal compounds may be used as commercial products.

### <Composition>

The composition of the invention comprises a metal complex of the invention and a charge transporting organic compound.

The composition of the invention is, for example, a mixture of a charge transporting organic compound as a host compound, with a metal complex of the invention. The host compound may be a hitherto known low molecular host compound or a high molecular compound for metal complex phospholuminescent compounds. The charge transport property is preferably a property of transporting both positive holes and electrons, and depending on the property of the metal complex used, it may be a property of transporting primarily only one electrical charge.

When the EL element obtained using a composition of the invention exhibits polarized EL luminescence, it is necessary to orient the charge transporting organic compound used as the host compound in the composition of the invention, and the metal complex will have a matching orientation property. Consequently, the charge transporting organic compound preferably exhibits a liquid crystal phase, and the metal complex is preferably compatible with the liquid crystal phase of the charge transporting organic compound and exhibits a uniform phase. The metal complex of the invention also preferably exhibits a liquid crystal phase. The composition also preferably forms a uniform phase and exhibits a liquid crystal phase. The temperature range of the liquid crystal phase is preferably room temperature or higher, and more preferably the crystal-liquid crystal transition temperature is in the range of 50-250°C.
The charge transporting organic compound may be used alone or in combinations of two or more.

The charge transporting organic compound may be a low molecular compound or a high molecular compound, but from the viewpoint of luminous efficiency when used as a light emitting element, the element characteristics such as usable life and the film formability, the number-average molecular weight (or molecular weight) in terms of polystyrene is preferably at least 4 × 10² and less than 3 × 10³ and more preferably at least 4.8 × 10² and less than 3 × 10³, for a low molecular compound, and the number-average molecular weight in terms of polystyrene is preferably 3 × 10³ to 1 × 10⁸ and more preferably 1 × 10⁴ to 1 × 10⁶, for a high molecular compound.
As used herein, a "high molecular compound" is a compound with a number-average molecular weight (or molecular weight) of 3 × 10³ or greater, while a low molecular compound is a compound with a number-average molecular weight of less than 3 × 10³, in terms of polystyrene. Also, the charge transporting organic compound may be in the form of a dendrimer or oligomer, regardless of whether it is a high molecular compound or a low molecular compound by this definition.

Low molecular host compounds include the following compounds.

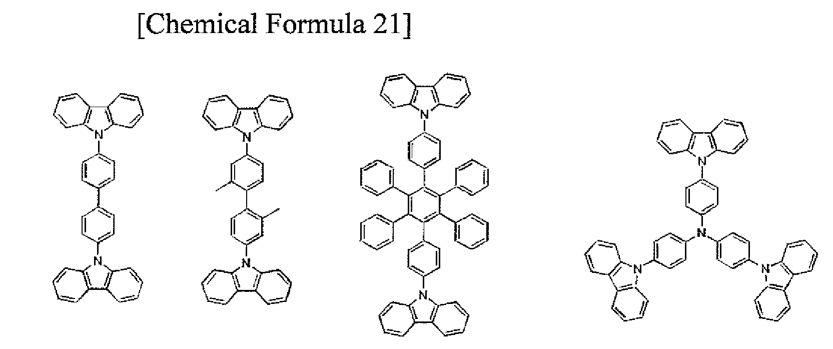

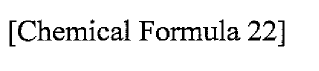

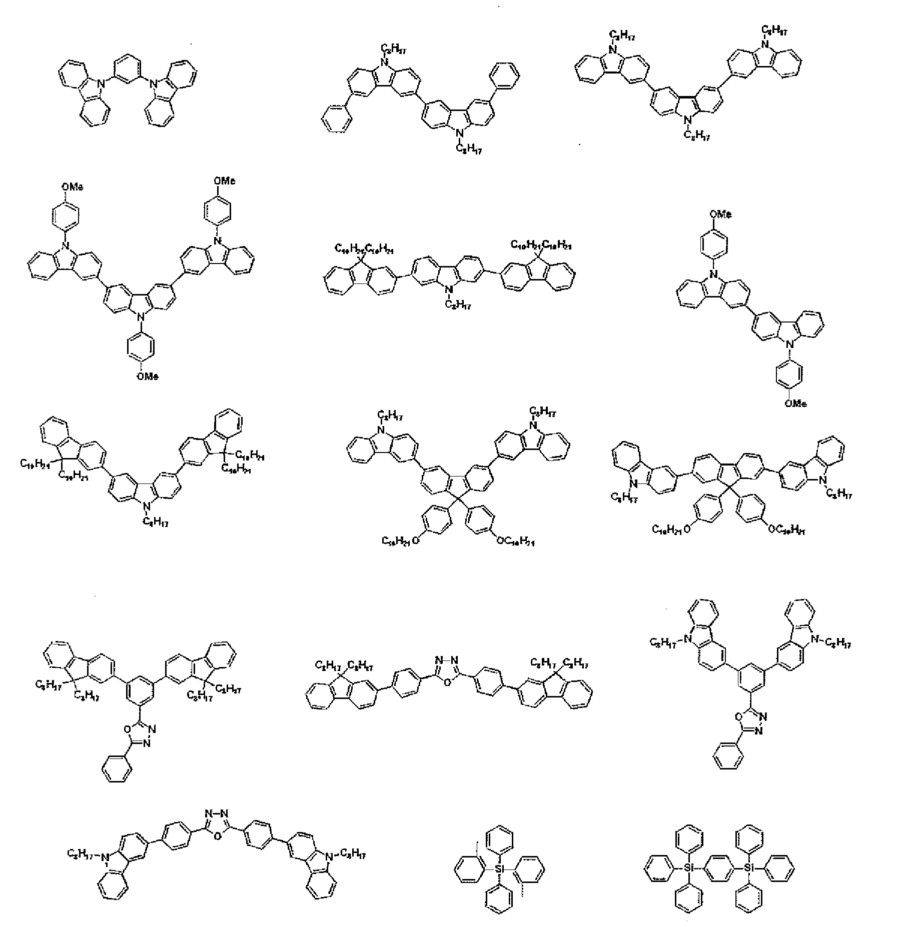

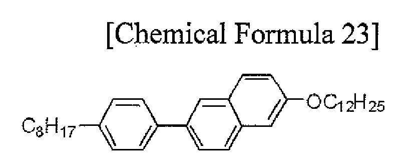

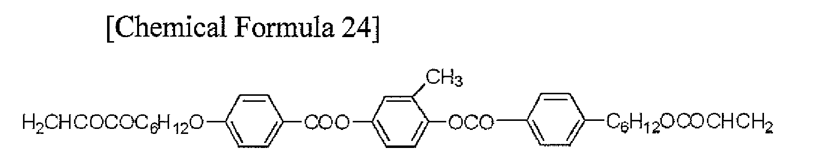

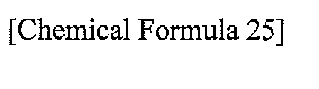

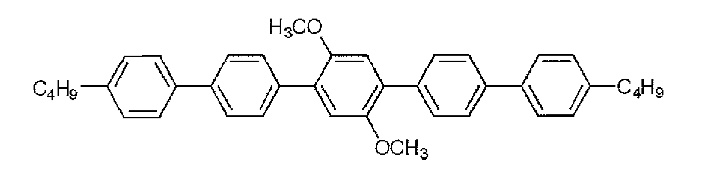

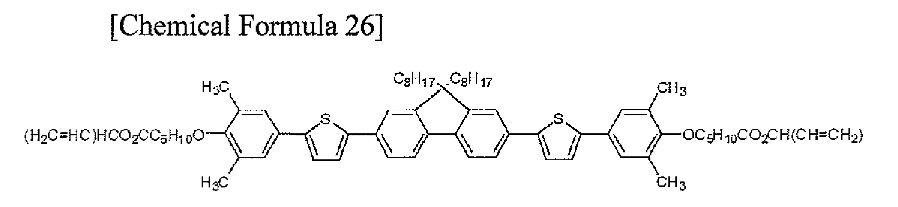

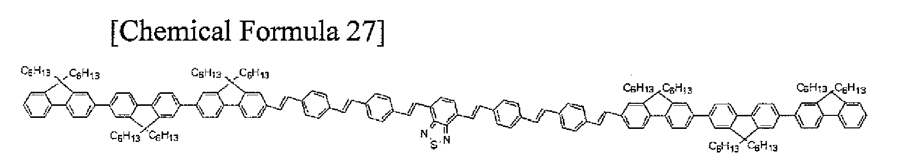

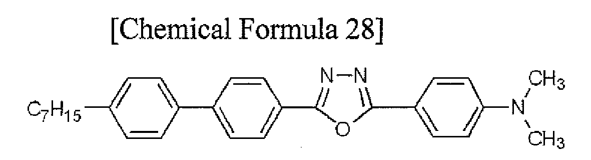

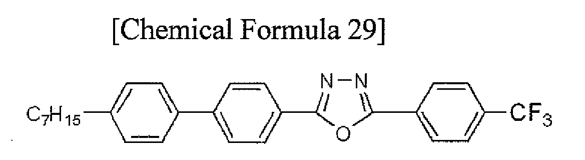

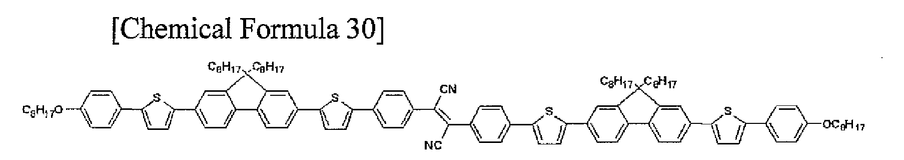

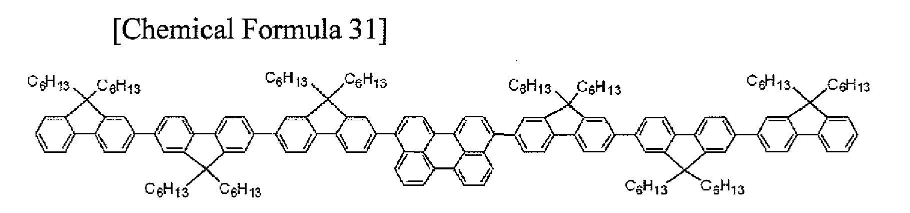

High molecular compounds may also be used as host compounds. Such high molecular compounds include nonconjugated high molecular compounds and conjugated high molecular compounds. A nonconjugated high molecular compound is a high molecular compound having a repeating unit such as a vinylene group or acrylate, while a conjugated high molecular compound is a polymer comprising an aromatic ring on the main chain.
Nonconjugated high molecular compounds include polyvinylcarbazole and the acrylate polymers mentioned in Japanese Unexamined Patent Application Publication No. 2003-133073, which include acrylate polymers comprising structures represented by any of the following formulas.

<Examples of acrylate polymers>

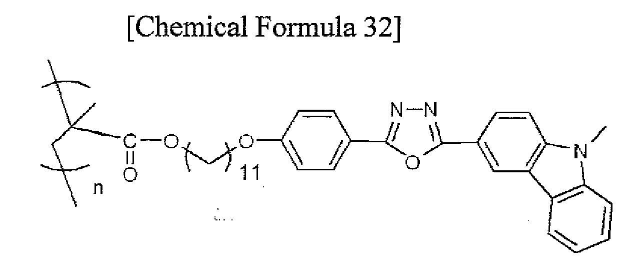

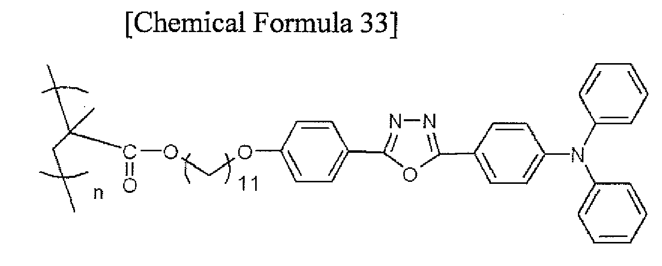

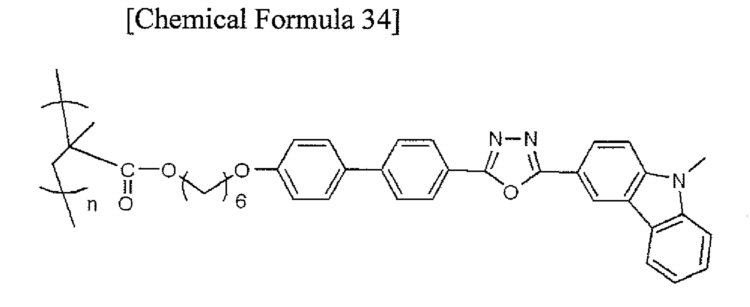

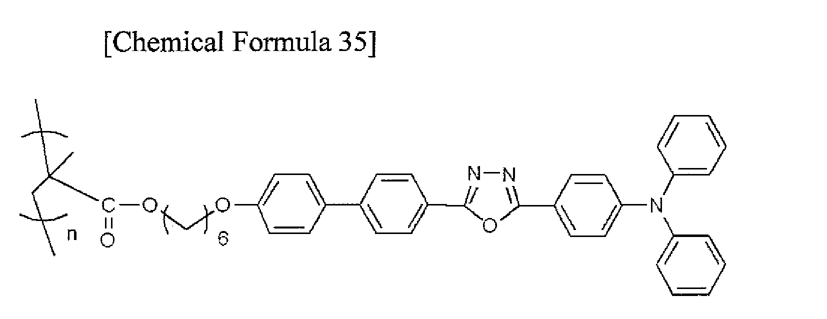

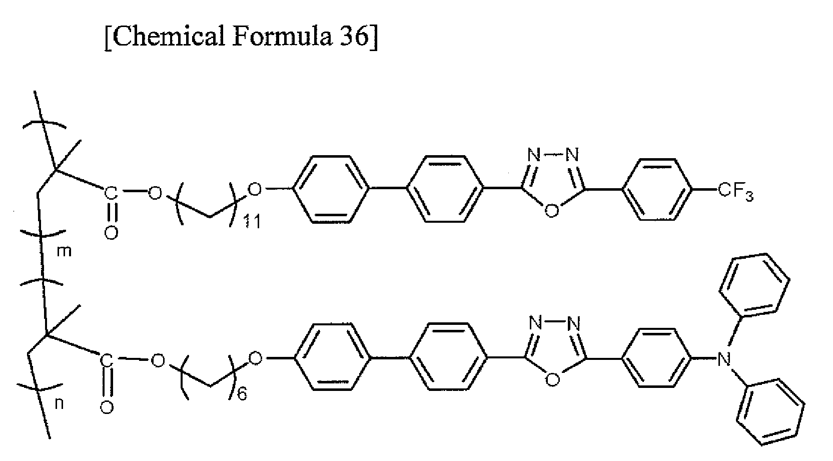

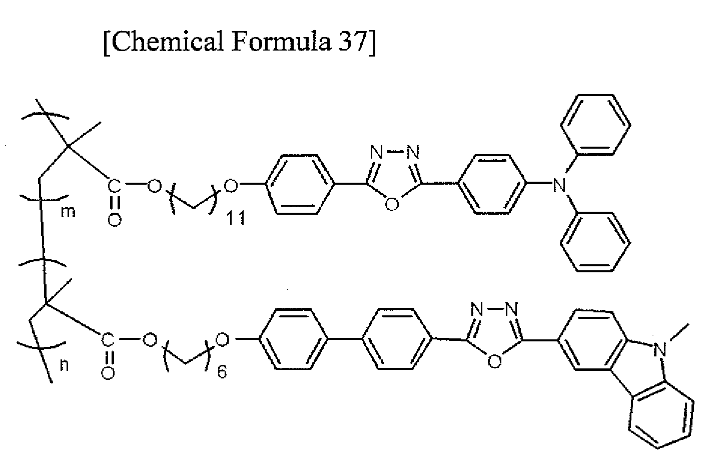

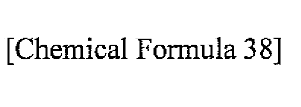

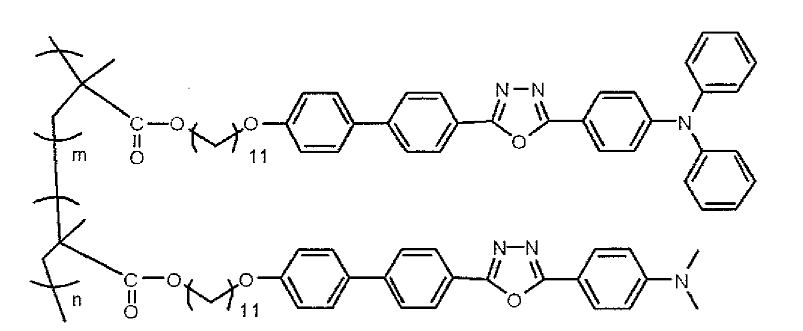

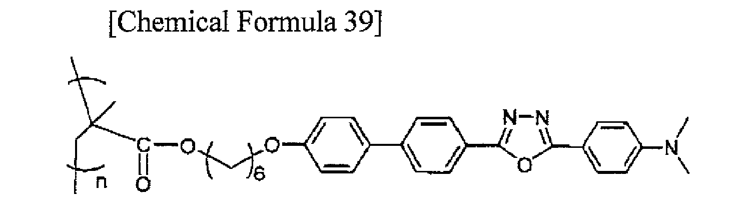

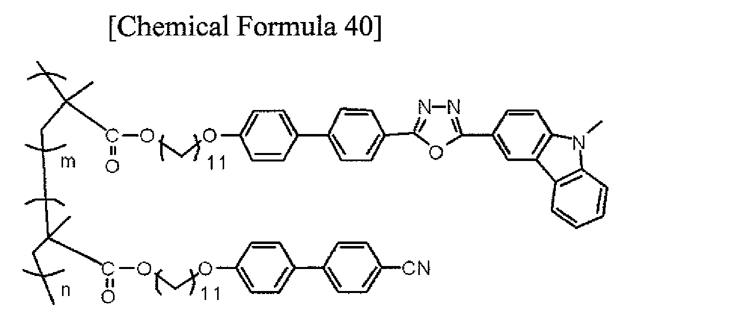

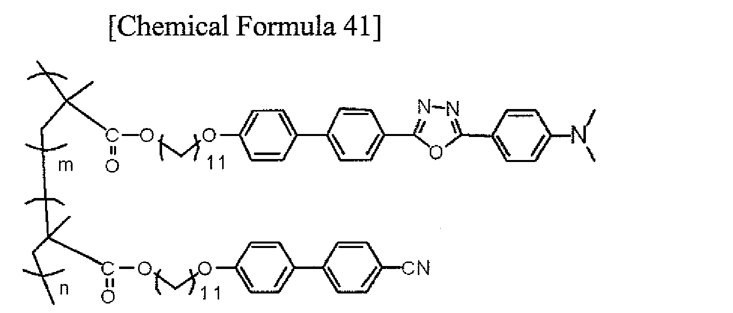

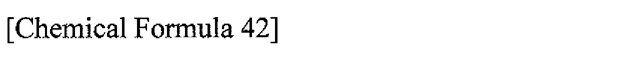

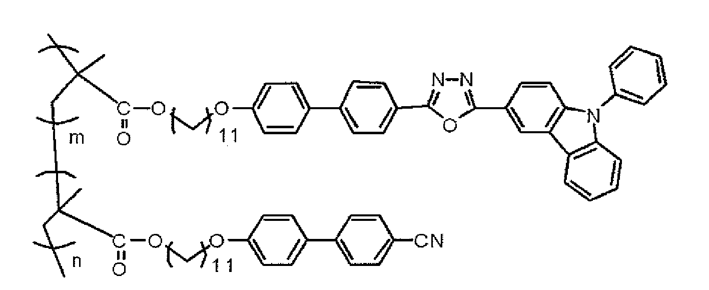

(In the formulas, n and m each independently represent the polymerization degree.)

The weight-average molecular weight of a nonconjugated high molecular compound used in the composition of the invention, in terms of polystyrene, is preferably 8 × 10³ to 1 × 10⁵ and even more preferably 1.8 × 10⁴ to 5 × 10⁴. The ratio of the weight-average molecular weight and number-average molecular weight, Mw/Mn, is preferably a smaller value.
The conjugated high molecular compound may be a polymer comprising an aromatic ring on the main chain, and it preferably comprises optionally substituted phenylene, optionally substituted fluorenediyl, optionally substituted dibenzothiophenediyl, optionally substituted dibenzofurandiyl, optionally substituted dibenzosiloldiyl or the like as a repeating unit on the main chain, or is a copolymer with such units. Such conjugated high molecular compounds include high molecular compounds having an optionally substituted benzene ring as a partial structure, and the high molecular compounds mentioned in Japanese Unexamined Patent Application Publication No. 2003-231741, Japanese Unexamined Patent Application Publication No. 2004-059899, Japanese Unexamined Patent Application Publication No. 2004-002654, Japanese Unexamined Patent Application Publication No. 2004-292546, US5708130, WO99/54385 WO0046321, WO02/077060, "Organic EL displays" (Tokito, S., Adachi, C., Murata, H., Ohmsha, Ltd.) p.111, Periodical: Displays (vol.9, No.9, 2002), p.47-51, examples of which include high molecular compounds comprising repeating units represented by the following formulas.

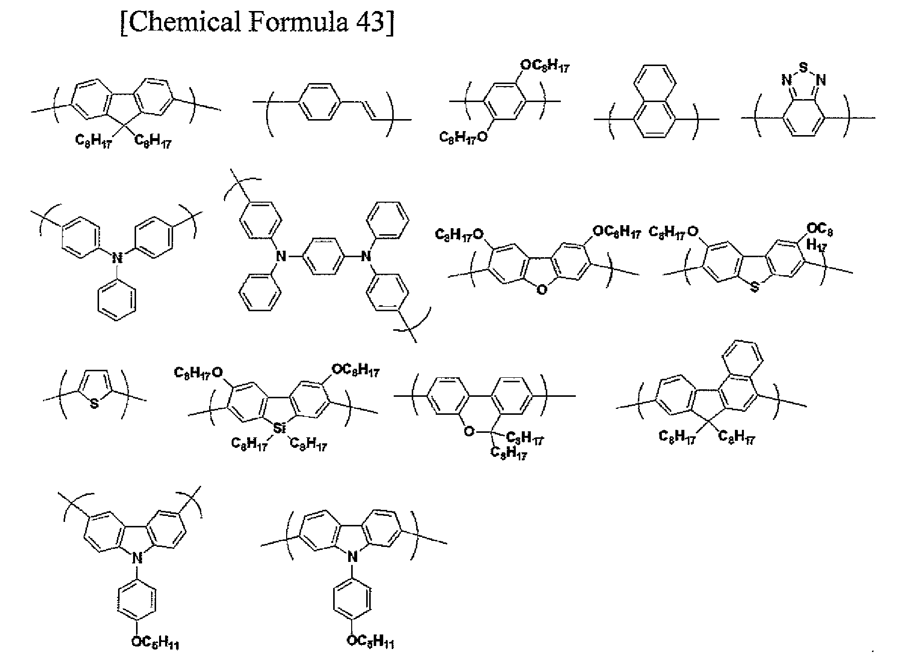

The high molecular compound used as a host compound may be a copolymer comprising a repeating unit represented by any of the formulas shown above. These host compounds may be used alone or in combinations of two or more.

The number-average molecular weight of a conjugated high molecular compound used in the composition of the invention, in terms of polystyrene, is preferably 3 × 10³ to 1 × 10⁸ and even more preferably 1 × 10⁴ to 1 × 10⁶. The weight-average molecular weight in terms of polystyrene is 3 × 10³ to 1 × 10⁸ and preferably 5 × 10⁴ to 5 × 10⁶.

When the charge transporting organic compound is a high molecular-compound, it may be a random copolymer, block copolymer or graft copolymer, or it may be a high molecular compound having such intermediate structures, for example, a block-type random copolymer. These charge transporting organic compounds include those having branches in the main chain, with 3 or more end portions, and dendrimers.

The minimum triplet excitation energy of the host compound (TH) and the minimum triplet excitation energy of the metal complex of the invention (TM) preferably satisfy the following relationship: TH > TM - 0.2 (eV). The values for the minimum triplet excitation energy and minimum triplet excitation energy can be obtained by computational scientific methods, and for example, they may be determined by the computational method described in Japanese Unexamined Patent Application Publication No. 2007-106990.

The charge transporting organic compound used for the invention preferably exhibits a liquid crystal phase, when polarized EL luminescence is to be obtained. The liquid crystal phase exhibited by the charge transporting organic compound is preferably a nematic phase. The temperature range of the liquid crystal phase is preferably a higher temperature than room temperature, and preferably it has a crystal-liquid crystal transition point in the range of 60°C-130°C.

The metal complex in the composition of the invention is present at usually 0.01-80 parts by weight and preferably 0.1-60 parts by weight, with 100 parts by weight as the amount of charge transporting organic compound, although this will differ depending on the type of charge transporting organic compound with which it is combined, and the desired properties. The metal complex may be used alone, or in a combination of two or more.

The composition of the invention may comprise a solvent or dispersing medium. The solvent or dispersing medium used may be selected from among known stable solvents that uniformly dissolve or disperse the film component. Solvents include hydrocarbon chloride-based solvents (chloroform, methylene chloride, 1,2-dichloroethane, 1,1,2-trichloroethane, chlorobenzene, o-dichlorobenzene and the like), ether-based solvents (tetrahydrofuran, dioxane and the like), aromatic hydrocarbon-based solvents (benzene, toluene, xylene and the like), aliphatic hydrocarbon-based solvents (cyclohexane, methylcyclohexane, n-pentane, n-hexane, n-heptane, n-octane, n-nonane, n-decane and the like), ketone-based solvents (acetone, methyl ethyl ketone, cyclohexanone and the like), ester-based solvents (ethyl acetate, butyl acetate, ethyl cellosolve acetate and the like), polyhydric alcohols and their derivatives (ethylene glycol, ethyleneglycol monobutyl ether, ethyleneglycol monoethyl ether, ethyleneglycol monomethyl ether, dimethoxyethane, propylene glycol, diethoxymethane, triethyleneglycol monoethyl ether, glycerin, 1,2-hexanediol and the like), alcohol-based solvents (methanol, ethanol, propanol, isopropanol, cyclohexanol and the like), sulfoxide-based solvents (dimethyl sulfoxide and the like), and amide-based solvents (N-methyl-2-pyrrolidone, N,N-dimethylformamide and the like). These solvents may be used alone or in combinations of two or more.

When a composition comprising such a solvent or dispersing medium is to be applied in an ink jet printing method, the composition may contain other additives to obtain a satisfactory discharge property for the composition, and its reproducibility. Other additives include high boiling point solvents (anisole, bicyclohexylbenzene and the like) to minimize evaporation from the nozzle. The composition comprising the solvent or dispersing medium preferably has a 25°C viscosity of 1-100 mPa·s.

### <Film employing a composition of the invention>

A film employing a metal complex or composition of the invention will now be described.
The method of forming a film of the invention may be vacuum vapor deposition (resistance heating vapor deposition, electron beam methods and the like), sputtering, LB, molecular stacking, or a coating method (casting, spin coating, bar coating, blade coating, roll coating, gravure printing, screen printing, ink jet printing or the like). Coating methods are preferred among these from the viewpoint of allowing the production process to be simplified. The solution to be used for film formation by a coating method is a composition comprising the aforementioned solvent or dispersing medium. The metal complex of the invention, and the composition comprising it, are advantageous in that they allow a coating method (wet process) to be applied.

### <Orientation treatment method>

The film of the invention exhibits polarized luminescence due to orientation treatment during the film formation step.
The film of the invention is preferably subjected to orientation treatment in at least one direction within the plane.

The orientation treatment method used may be a known method such as described in Japanese Patent Public Inspection HEI No. 10-508979, Japanese Unexamined Patent Application Publication No. 2003-133073 or Japanese Unexamined Patent Application Publication No. 2004-31210. For example, a film may be formed first on a substrate and rubbed in one direction, after which a film composed of the composition of the invention may be formed, thereby orienting it. The rubbing may also be followed by annealing treatment to obtain a film with a higher degree of orientation. Here, rubbing refers to treatment by rubbing with a film or sheet, and it is a method commonly used for production of liquid crystal displays. The film for rubbing may be a polyimide film, or PEDOT (polyethylenedioxythiophene) that functions as a conductive or positive hole injection layer. Annealing treatment involves raising the temperature of the luminescent layer to the liquid crystal phase temperature or to an isotropic phase, after formation of the luminescent layer, and slowly cooling it.
The film obtained using the metal complex of the invention or a composition comprising it, may also be oriented by a friction method involving direct rubbing with a fabric or Teflon^{R} block.

When a charge transporting organic compound that exhibits a liquid crystal phase and has a polymerizable group is used as the host compound in the composition of the invention, the orientation is fixed by forming a luminescent layer made of the aforementioned composition on a film subjected to the orientation treatment described above, and then raising the temperature to the liquid crystal phase temperature or to an isotropic phase, slowly cooling it for orientation, and subsequently accomplishing polymerization and high molecularization with photoirradiation or the like.

### <Light emitting element>

A light emitting element of the invention is obtained using a film comprising a metal complex of the invention as the luminescent layer. Examples of such light emitting elements include light emitting elements having electrodes including an anode and cathode, and a layer comprising the aforementioned metal complex (optionally included as the aforementioned composition) formed between the electrodes. The composition of the invention may be used to form a film, and the film used to fabricate a light emitting element of the invention.

The light emitting element of the invention has a pair of electrodes including an anode and cathode, and a film with a single layer (monolayer) or multiple layers (multilayer) comprising at least a luminescent layer held between the electrodes. At least one of the layers of the film comprises a metal complex of the invention. The metal complex will in most cases be included as a composition with the charge transporting organic compound as the host compound, and the total content of the metal complex and the charge transporting organic compound in the film will usually be 0.1-100 wt%, preferably 0.1-30 wt%, more preferably 0.5-30 wt% and most preferably 1-30 wt%, with respect to the weight of the entire luminescent layer.

When the light emitting element of the invention is a monolayer, the sole film is the luminescent layer and the luminescent layer contains the metal complex. When the light emitting element of the invention is a multilayer, it may have one of the following laminar structures, for example, with each luminescent layer comprising the metal complex.
(a) Anode/positive hole injection layer (positive hole transport layer)/luminescent layer/cathode
(b) Anode/luminescent layer/electron injection layer (electron transport layer)/cathode
(c) Anode/positive hole injection layer (positive hole transport layer)/luminescent layer/electron injection layer (electron transport layer)/cathode

The anode of the light emitting element of the invention supplies positive holes to the positive hole injection layer, positive hole transport layer and luminescent layer, and it is effective for it to have a work function of 4.5 eV or greater. As the anode material there may be used a metal, alloy, metal oxide or electrically conductive compound, or a mixture thereof Specifically, it may be a conductive metal oxide such as tin oxide, zinc oxide, indium oxide or indium tin oxide (ITO), a metal such as gold, silver, chromium or nickel, or a mixture or laminate of these conductive metal oxides and metals, an inorganic conductive substance such as copper iodide or copper sulfide, an organic conducting material such as a polyaniline, polythiophene (PEDOT or the like) or polypyrrole, or a laminate of these with ITO.

The cathode of the light emitting element of the invention supplies electrons to the electron injection layer, electron transport layer and luminescent layer. As the cathode material there may be used a metal, alloy, metal halide, metal oxide, electrically conductive compound, or a mixture thereof. Cathode materials include alkali metals (lithium, sodium, potassium and the like) and their fluorides and oxides, alkaline earth metals (magnesium, calcium, barium, cesium and the like) and their fluorides and oxides, gold, silver, lead, aluminum, alloys and blend alloys (sodium-potassium alloy, sodium-potassium blend alloy, lithium-aluminum alloy, lithium-aluminum blend alloy, magnesium-silver alloy, magnesium-silver blend alloy, and the like), and rare earth metals (indium, ytterbium and the like).

The positive hole injection layer and positive hole transport layer of the light emitting element of the invention may have a function of injecting positive holes from the anode, a function of transporting positive holes, or a function of serving as a barrier to electrons injected from the cathode. Such a layer material may be a carbazole derivative, triazole derivative, oxazole derivative, oxadiazole derivative, imidazole derivative, polyarylalkane derivative, pyrazoline derivative, pyrazolone derivative, phenylenediamine derivative, arylamine derivative, amino-substituted chalcone derivative, styrylanthracene derivative, fluorenone derivative, hydrazone derivative, stilbene derivative, silazane derivative, aromatic tertiary amine compound, styrylamine compound, aromatic dimethylidene compound, porphyrin-based compound, polysilane-based compound, poly (N-vinylcarbazole) derivative or organosilane derivative, or a polymer comprising the foregoing. It may also be a conductive polymer oligomer, such as an aniline-based copolymer, thiophene oligomer or polythiophene. These materials may be used alone as single components, or as multiple components in combination. Also, the positive hole injection layer and positive hole transport layer may have a monolayer structure comprising one or more of the aforementioned materials, or it may have a multilayer structure comprising multiple layers of the same composition or different compositions.

The electron injection layer and electron transport layer of the light emitting element of the invention may have a function of injecting electrons from the cathode, a function of transporting electrons, or a function of serving as a barrier to positive holes injected from the anode. The materials for such layers may be various types of metal complexes including metal complexes of triazole derivatives, oxazole derivatives, oxadiazole derivatives, imidazole derivatives, fluorenone derivatives, anthraquinodimethane derivatives, anthrone derivatives, diphenylquinone derivatives, thiopyran dioxide derivatives, carbodiimide derivatives, fluorenylidenemethane derivatives, distyrylpyrazine derivatives, tetracarboxylic anhydrides of aromatic rings such as naphthalene and perylene, phthalocyanine derivatives or 8-quinolinol derivatives, metal phthalocyanines, and metal complexes whose ligands are benzooxazole or benzothiazole, as well as organosilane derivatives. Also, the electron injection layer and electron transport layer may have a monolayer structure comprising one or more of the aforementioned materials, or it may have a multilayer structure comprising multiple layers of the same composition or different compositions.

In a light emitting element of the invention, the materials used for the electron injection layer and electron transport layer may also be insulator or semiconductor inorganic compounds. If the electron injection layer and electron transport layer are formed with an insulator or semiconductor, it is possible to effectively prevent leakage of current and increase the electron injection property. Such an insulator may be at least one metal compound selected from the group consisting of alkali metal chalcogenides, alkaline earth metal chalcogenides, alkali metal halides and alkaline earth metal halides. Preferred alkali metal chalcogenides include CaO, BaO, SrO, BeO, BaS and CaSe. Semiconductors used to form the electron injection layer and electron transport layer may be oxides, nitrides or oxynitrides comprising at least one element selected from the group consisting of Ba, Ca, Sr, Yb, Al, Ga, In, Li, Na, Cd, Mg, Si, Ta, Sb and Zn. These oxides, nitrides and oxynitrides may be used alone or in combinations of 2 or more.

A reducing dopant may also be added at the interface region with the film adjacent to the cathode. Preferred as reducing dopants are one or more compounds selected from the group consisting of alkali metals, alkaline earth metal oxides, alkaline earth metals, rare earth metals, alkali metal oxides, alkali metal halides, alkaline earth metal oxides, alkaline earth metal halides, rare earth metal oxides, rare earth metal halides, alkali metal complexes, alkaline earth metal complexes and rare earth metal complexes.

The luminescent layer of the light emitting element of the invention has the function of allowing injection of positive holes from the anode or positive hole injection layer and allowing injection of electrons from the cathode or electron injection layer, during application of voltage, the function of causing migration of injected electrical charge (electrons and positive holes) by electric field force, and the function of providing a site for recombination between electrons and positive holes, leading to luminescence. A metal complex of the invention or a composition of the invention is preferably used in the luminescent layer of the light emitting element of the invention.

The method of forming each layer in the light emitting element of the invention may be vacuum vapor deposition (resistance heating vapor deposition, electron beam methods and the like), sputtering, LB, molecular stacking, or a coating method (casting, spin coating, bar coating, blade coating, roll coating, gravure printing, screen printing, ink jet printing or the like). Coating methods are preferred among these from the viewpoint of allowing the production process to be simplified. In the aforementioned coating methods, formation may be accomplished by dissolving the metal complex and charge transporting organic compound in a solvent to prepare a coating solution, and coating and drying the coating solution on the desired layer (or electrode).

Polarized luminescence can be obtained in the light emitting element of the invention by carrying out the orientation treatment mentioned above during formation of the luminescent layer.

The preferred film thickness of each layer of the light emitting element of the invention will differ depending on the type of material and the laminar structure, but an excessively small film thickness will generally tend to result in defects such as pinholes, while an excessively large thickness will require a high applied voltage and will reduce the luminous efficiency, and it is therefore preferably 30 nm-1 µm.

Examples of usage of the light emitting element of the invention include planar light sources, lighting fixtures, light sources, sign light sources, backlight light sources, display units and printer heads. A segment-type or dot matrix-type construction may be selected for the display unit, using known driving technology, driving circuits and the like.

### Examples

Examples will now be explained for more detailed explanation of the invention, with the understanding that the invention is not limited by the examples.

### [Example 1]

<Synthesis of metal complex (MC-1)>

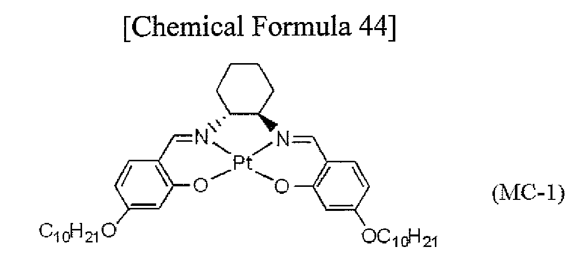

### (1) Synthesis of 4-(decyloxy)salicylaldehyde:

To 70 ml of DMF there were added 2,4-dihydroxybenzaldehyde (6.94 g), 1-bromodecane (11.2 g), potassium hydrogencarbonate (5.16 g) and a trace amount of potassium iodide. The obtained mixture was increased in temperature to 135°C and stirred for 4 hours. The mixture was cooled to room temperature and then poured into 1N-hydrochloric acid (500 ml). The reaction product was extracted with chloroform and the solvent of the organic layer was distilled off. The residue was purified by silica gel-packed column chromatography (developing solution: hexane:ethyl acetate = 9:1 (volume ratio)) to obtain 4-(decyloxy)salicylaldehyde as a colorless oil. The yield was 10.4 g (72%).
· Measured values: ¹H NMR (300 MHz, CDCl₃, δ): 0.88 (t, J = 6.8 Hz, 3H), 1.27-1.79 (m, 16H), 4.00 (t, 2H), 6.41 (d, 1H), 6.51 (dd, 1H), 7.40 (d, 1H), 9.70 (s, 1H), 11.5 (s, 1H).

### (2) Synthesis of N,N'-bis(4-decyloxysalicylidene)-(1R, 2R)-(-)cyclohexanediamine:

After mixing the 4-(decyloxy)salicylaldehyde obtained in the above (1) (4.12 g), (1R, 2R)-diaminocyclohexane (0.94 g) and absolute ethanol (30 ml), the obtained solution was stirred at 50°C for 14 hours. The solution was cooled to room temperature and the solvent was removed.
The residue was purified by silica gel-packed column chromatography (developing solution: toluene: THF = 15:1 (volume ratio)) and then concentrated. The obtained product was recrystallized from methanol to obtain the target compound N,N-bis(4-decyloxysalicylidene)-(1R, 2R)-(-)cyclohexanediamine. The yield was 3.02 g (65%).
· Measured values: ¹H NMR (δ, CDCl₃): 0.79 (t, J = 6.6 Hz, 6H), 0.99-1.87 (m, 40H), 3.21 (s, 2H), 3.82 (t, 4H), 6.18-6.38 (4H, m), 6.93 (2H, d), 8.07 (2H, s), 13.8 (2H, s)
· Calculated: C₄₀H₆₂N₂O₄ (635)
· MS-FAB⁺(m-NBA): m/z 635 [M]⁺.

### (3) Synthesis of metal complex (MC-1) [N,N'-bis(4-decyloxysalicylidene)-(1R, 2R)-(-)cyclohexane diaminate platinum(II) complex]

After suspending N,N'-bis(4-decyloxysalicylidene)-(1R, 2R)-(-)cyclohexanediamine (2.00 g) in dichlorobis(dimethyl sulfoxide)platinum (II) (1.36 g) and anhydrous acetonitrile (20 ml), the suspension was vigorously stirred at 50°C for 6 hours. The solvent was then removed by evaporation under reduced pressure and recrystallized from tetrahydrofuran/ethyl acetate to obtain 0.65 g of the target complex N,N'-bis(4-decyloxysalicylidene)-(1R, 2R)-(-)cyclohexane diaminate platinum(II) (metal complex (MC-1)). The yield was 25%.
· Measured values: ¹H NMR (δ, CDCl₃): 0.89 (t, 6H), 0.95-1.76 (m, 40H), 3.50 (s, 2H), 3.93 (t, 4H), 6.22 (d, 2H), 6.70 (s, 2H), 7.11 (d, 2H), 7.75 (s, 2H). MS-FAB⁺(m-NBA): m/z 828 [M]⁺.

### [Example 2]

<Synthesis of metal complex (MC-2)>

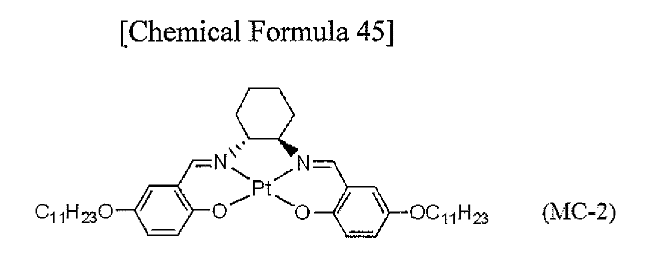

### (1) Synthesis of 4-(n-undecyloxy)phenol:

After dissolving hydroquinone (20 g) in a mixture of 1-bromoundecane (10.0 g) and methyl ethyl ketone (50 ml), a trace amount of potassium iodide and potassium carbonate (6.21 g) were slowly added. The obtained solution was then stirred under reflux for 24 hours. The solvent was removed by evaporation under reduced pressure, and the crude product was extracted with ethyl acetate and rinsed with 1N hydrochloric acid and water. The rinsed product was purified by silica gel-packed column chromatography (developing solution: chloroform) and recrystallized from a hexane/acetone mixture to obtain the target compound 4-(n-undecyloxy)phenol. The yield was 9.22 g (82%).
· Measured values: ¹H NMR (300 MHz, CDCl₃, δ): 0.88 (t, 3H), 1.27-1.79 (m, 18H), 3.89 (t, 2H), 4.48 (s, 1H), 6.73-6.91 (m, 4H).

### (2) Synthesis of 5-(undecyloxy)salicylaldehyde:

The 4-(undecyloxy)phenol obtained in the above (1) (10.0 g) was suspended in hexamethylenetetraamine (5.3 g) and trifluoroacetic acid (40 ml). The suspension was vigorously stirred at 100°C for 1 hour, and then further stirred at room temperature for 2 hours. To this there was added 4N-hydrochloric acid (40 ml), and the mixture was extracted with dichloroethane. The organic solvent was removed by evaporation under reduced pressure and the obtained black oil was purified by silica gel column chromatography (eluent: ethyl acetate:hexane = 1:9 (volume ratio)) to obtain the target compound 5-(undecyloxy)salicylaldehyde. The yield was 2.11 g (19%).

### (3) Synthesis of N,N'-bis(5-undecyloxysalicylidene)-(lR, 2R)-(-)cyclohexanediamine:

After mixing the 5-(undecyloxy)salicylaldehyde obtained in the above (2) (4.39 g), (1R, 2R)-1,2-cyclohexanediamine (0.94 g) and absolute ethanol, the solution was stirred at 50°C for 12 hours. The solution was restored to room temperature, the solvent was subsequently removed by evaporation, and the residue was purified by silica gel column chromatography (eluent: toluene: THF = 15:1 (volume ratio)) and the solution concentrated. The obtained reaction product was recrystallized from methanol to obtain 2.32 g of the target compound N,N'-bis(5-undecyloxysalicylidene)-(1R, 2R)-(-)cyclohexanediamine.
(Yield: 48%)
· Measured values: ¹H NMR (δ, CDCl₃): 0.79 (t, 6H), 0.91-1.95 (m, 44H), 3.22 (s, 2H), 3.92 (t, 4H), 6.13-6.29 (m, 4H), 7.01 (d, 2H), 8.06 (s, 2H), 13.6 (s, 2H)
· Calculated: C₄₂H₆₆N₂O₄ (663)
· MS-FAB⁺(m-NBA): m/z 663 [M]⁺.

### (4) Synthesis of metal complex (MC-2) [N,N'-bis(5-undecyloxysalicylidene)-(1R, 2R)-(-)cyclohexane diaminate platinum(II)]

The N,N'-bis(5-undecyloxysalicylidene)-(1R, 2R)-(-)cyclohexanediamine obtained in the above (3) (2.00 g) and dichlorobis(dimethyl sulfoxide)platinum(II) (1.36 g) were suspended in anhydrous acetonitrile (20 ml).
The obtained suspension was powerfully stirred at 50°C for 6 hours, and then the solvent was removed by evaporation under reduced pressure. Recrystallization was then performed from a tetrahydrofuran/ethyl acetate mixed solvent, to obtain 0.52 g of the target compound, N,N'-bis(5-undecyloxysalicylidene)-(1R, 2R)-(-)cyclohexane diaminate platinum(II) complex (metal complex (MC-2)). The yield was 20%.
· Measured values: ¹H NMR (δ, CDCl₃): 0.89 (t, 6H), 0.95-2.01 (44H, m), 3.23 (s, 2H), 3.97 (t, 4H), 6.25 (d, 2H), 6.69 (s, 2H), 7.41 (d, 2H), 7.78 (s, 2H).
   MS-FAB⁺(m-NBA): m/z 856 [M]⁺
· Calculated: C₄₂H₆₄N₂O₄Pt (856): C, 58.93; H, 7.54; N, 3.27; O, 7.48.
· Found: C, 58.65; H, 7.57; N, 3.25; O, 7.56.

### [Example 3]

### <Fabrication of EL element (A)>

A solution of poly(ethylenedioxythiophene)/polystyrenesulfonic acid (trade name: CLEVIOS P VP AI4083 by H.C. Starck) was used for film formation by spin coating to a thickness of 65 nm on a glass panel, which had an ITO film with a thickness of 45 nm formed thereon by sputtering, and the film was dried for 10 minutes at 200°C on a hot plate.
Next, the high molecular compound (I-1) described below was spin coated as a 0.8 wt% xylene solution, to form a film with a thickness of approximately 20 nm. It was then heat treated for 60 minutes at 180°C on a hot plate.

Next, a solution of the compound represented by the following formula:

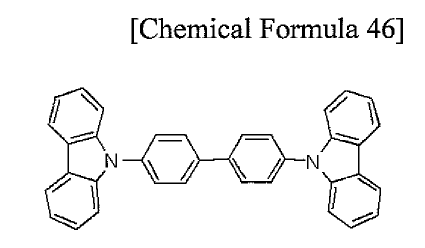

(HL-1) (product of Tokyo Chemical Industry Co., Ltd.) dissolved in a chloroform solvent to a concentration of 0.8 wt%, and a solution of metal complex (MC-1) dissolved in a chloroform solvent to a concentration of 0.8 wt%, were combined at a weight ratio of 90:10 to prepare a composition (hereunder referred to as "composition 1"). Composition 1 was spin coated to form a film, at a rotational speed of 3500 rpm. The film thickness was approximately 80 nm. This was subjected to drying for 10 minutes at 60°C under a nitrogen gas atmosphere, and then to vapor deposition with barium to approximately 5 nm and then aluminum to approximately 60 nm as a cathode, to fabricate an EL element (A). Vapor deposition of the metals was initiated after the degree of vacuum reached at least 1 × 10⁻⁴ Pa.
Upon application of a voltage to the obtained EL element (A), EL luminescence was obtained from the EL element (A), having a peak at 530 nm due to the metal complex (MC-1), and the maximum luminous efficiency was 5.1 cd/A.

### <Fabrication of EL element (D)>

A solution of HL-1 dissolved in a chloroform solvent to a concentration of 0.8 wt% and a solution of metal complex (MC-2) dissolved in a chloroform solvent to a concentration of 0.8 wt%, were combined at a weight ratio of 90:10 to prepare a composition (hereunder referred to as "composition 4"). EL element (D) was fabricated by the same method as for fabrication of EL element (A), except that composition 4 was used instead of composition 1. Upon application of a voltage to the obtained EL element (D), EL luminescence was obtained from the EL element (D), having a peak at 610 nm due to the metal complex (MC-2), and the maximum luminous efficiency was 1.7 cd/A.

### <Synthesis of high molecular compound (I-1)>

High molecular compound (I-1) was synthesized in the following manner.
To a Dimroth-connected flask there were added 5.25 g (9.9 mmol) of compound A represented by the following formula:

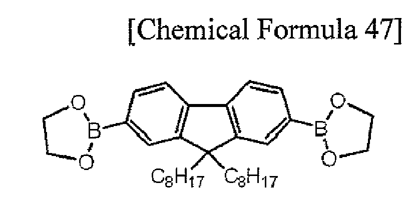

4.55 g (9.9 mmol) of compound B represented by the following formula:

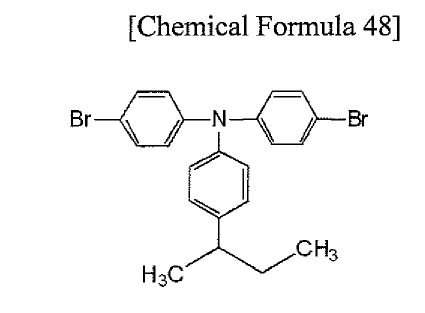

0.91 g of methyltrioctylammonium chloride (trade name: Aliquat 336, product of Aldrich Co.) and 69 ml of toluene, to obtain a monomer solution. The monomer solution was heated under a nitrogen atmosphere, and then 2 mg of palladium acetate and 15 mg of tris(2-methylphenyl)phosphine were added at 80°C. After then pouring 9.8 g of 17.5 wt% aqueous sodium carbonate into the obtained monomer solution, the mixture was stirred at 110°C for 19 hours. Next, 121 mg of phenylboric acid dissolved in 1.6 ml of toluene was added thereto and the mixture was stirred at 105°C for 1 hour.

The organic layer and aqueous layer were separated, and then 300 ml of toluene was added to the organic layer. The organic layer was washed with 40 ml of a 3 wt% acetic acid aqueous solution (2 times) and with 100 ml of ion-exchanged water (once), and separated from the aqueous layer. Next, 0.44 g of sodium N,N-diethyldithiocarbamate trihydrate and 12 ml of toluene were added to the organic layer, and the mixture was stirred at 65°C for 4 hours.
The obtained toluene solution of the reaction product was passed through a silica gel/alumina column that had been previously passed through with toluene, and the obtained solution was dropped into 1400 ml of methanol, producing a precipitate, and the precipitate was filtered and dried to obtain a solid. The solid was dissolved in 400 ml of toluene and dropped into 1400 ml of methanol, producing a precipitate, and the precipitate was filtered and dried to obtain 6.33 g of a high molecular compound (I-1). The number-average molecular weight Mn of the high molecular compound (I-1) in terms of polystyrene was 8.8 × 10⁴, and the weight-average molecular weight Mw in terms of polystyrene was 3.2 × 10⁵, as measured under [Analysis conditions 1] described below.
Based on the charged starting materials, the high molecular compound (I-1) is inferred to be a polymer comprising a repeating unit represented by the following formula: 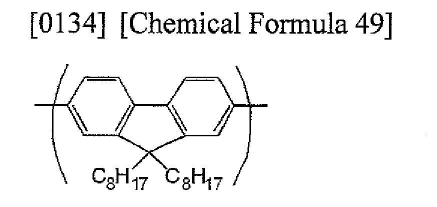 and a repeating unit represented by the following formula:

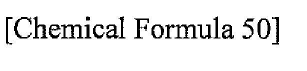

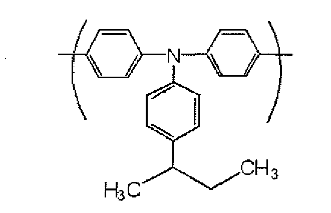

at a molar ratio of 1:1.
The number-average molecular weight and weight-average molecular weight of the high molecular compound (polymer) in terms of polystyrene were determined using size-exclusion chromatography (SEC) (LC-10Avp trade name of Shimadzu Corp.). The SEC analysis conditions used were according to the method described under [Analysis conditions 1].

### [Analysis conditions 1]

The high molecular compound (polymer) to be measured was dissolved in tetrahydrofuran to a concentration of about 0.05 wt% and 50 µL thereof was injected into the SEC apparatus. The SEC mobile phase was tetrahydrofuran, and the flow rate was 0.6 mL/min. The columns used were two TSKgel SuperHM-H (Tosoh Corp.) columns and one TSKgel SuperH2000 (Tosoh Corp.) column, connected in series. The detector used was a differential refractometer (trade name: RID-10A, product of Shimadzu Corp.).

The LC-MS measurement was conducted by the following method. The measuring sample was dissolved in chloroform or tetrahydrofuran to a concentration of about 2 mg/mL, and approximately 1 µL was injected into an LC-MS device (trade name: 1100LCMSD by Agilent Technologies). The LC-MS moving bed was used while varying the proportion of ion-exchanged water containing approximately 0.1 wt% added acetic acid, and acetonitrile containing approximately 0.1 wt% added acetic acid, with flow at a flow rate of 0.2 mL/min. The column used was an L-column 2 ODS (3 µm) (product of Chemicals Evaluation and Research Institute, Japan, inner diameter: 2.1 mm, length: 100 mm, particle size: 3 µm).
The NMR measurement was conducted by the following method. After dissolving 5-10 mg of the measuring sample in approximately 0.5 mL of heavy chloroform or heavy dimethyl sulfoxide, measurement was conducted using an NMR apparatus (trade name MERCURY 300 by Varian, Inc.).

### [Example 4]

### <Fabrication of EL element (B)>

A solution of poly(ethylenedioxythiophene)/polystyrenesulfonic acid (trade name: CLEVIOS P VP AI4083 by H.C. Starck) was used for film formation by spin coating to a thickness of 65 nm on a glass panel, which had an ITO film with a thickness of 45 nm formed thereon by sputtering, and the film was dried for 10 minutes at 200°C on a hot plate. The obtained substrate was returned to room temperature, and the substrate surface was rubbed.

A solution of the high molecular compound (H-1) described hereunder, dissolved in a 1,1,2,2-tetrachloroethane solvent to a concentration of 2.0 wt%, and a solution of metal complex (MC-1) dissolved in a 1,1,2,2-tetrachloroethane solvent to a concentration of 2.0 wt%, were then combined at a weight ratio of 90:10 to prepare a composition (hereunder referred to as "composition 2"). Composition 2 was spin coated to form a film, at a rotational speed of 1600 rpm. The film thickness was approximately 80 nm. This was heated with a hot plate at 160°C for 2 hours under a nitrogen atmosphere, and then immediately cooled to room temperature.

· High molecular compound (H-1)

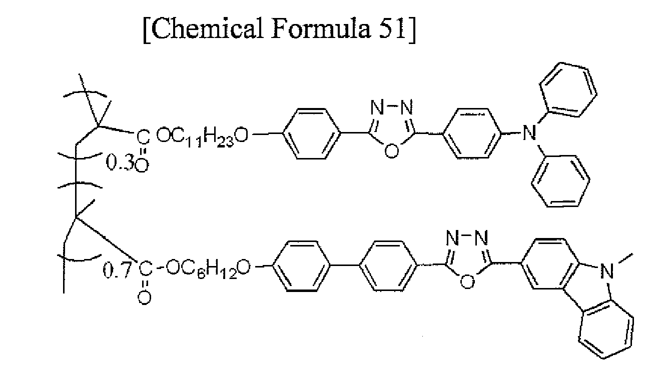

(The subscripts beside parentheses in the formula indicate the molar ratios of each repeating unit.)

The substrate that had been cooled to room temperature was transferred to a vapor deposition apparatus, and vapor deposited with barium to a thickness of about 5 nm and then with aluminum to a thickness of about 60 nm, as a cathode, to fabricate EL element (B). Vapor deposition of the metals was initiated after the degree of vacuum reached at least 1 × 10⁻⁴ Pa.
Upon application of a voltage to the obtained EL element (B), EL luminescence was obtained from the EL element (B), having a peak at 580 nm due to the metal complex (MC-1), and the maximum luminous efficiency was 0.45 cd/A. The EL luminescence was polarized luminescence in the direction parallel to the rubbing direction, and the degree of polarization was 11 at 580 nm. Measurement of the degree of polarization was accomplished in the following manner. Specifically, a fluorescence spectrophotometer (trade name: FP-6500 by JASCO Corp.) was used to measure the luminescence intensity (L1) with the polarizing plate set in front of the detector and the EL luminescence emitting element set with the absorption axis of the polarizing plate parallel to the rubbing direction, and the luminescence intensity (L2) with the same set perpendicular, and L2/L1 was recorded as the degree of polarization.

### <Synthesis of high molecular compound (H-1)>

High molecular compound (H-1) was synthesized in the following manner.

· Synthesis of monomer 1: [2-{4'-(6-methacryloyloxyhexyloxy)biphenyl-4-yl}-5-(9-methylcarbazo 1-3-yl)-1,3,4-oxadiazole]

After dissolving 17 g (79.5 mmol) of 4-(4-hydroxyphenyl)benzoic acid and 16.4 g (91 mmol) of 6-bromohexanol in 200 ml of ethanol, one spatula of potassium iodide was added, and the mixture was heated at 60°C and stirred. Also, a solution of 8 g (140 mmol) of potassium hydroxide in 20 ml of ethanol was slowly added dropwise, and the mixture was heated and stirred at 90°C for 10 hours. Upon completion of the reaction, the solvent was distilled off under reduced pressure, and then 500 ml of water was added to the reaction mixture, the pH was adjusted, and the precipitate was filtered to obtain a white solid. This was dried under reduced pressure, and then the recovered white solid was washed with methanol to obtain 10.6 g (33.5 mmol) of 4-(6-hydroxyhexyloxy)biphenyl-4'-carboxylic acid as a white powder.
(Yield: 42%)
· Measured values: ¹H-NMR (δ, DMSO-d₆): 1.40-1.87 (8H, m), 4.10 (2H, t), 4.22 (2H, t), 7.12 (2H, d), 7.74 (2H, d), 7.81 (2H, d), 8.09 (2H, d), 10.7 (1H, s)
· Calculated: C₁₉H₂₃O₄ (315): C, 72.36%; H, 7.35%; O, 20.29%
· Found: C, 72.70%; H, 6.90%; O, 20.40%

After adding 100 ml of 1,4-dioxane to 8.2 g (26 mmol) of 4-(6-hydroxyhexyloxy)biphenyl-4'-carboxylic acid and 5.5 ml of triethylamine, the mixture was stirred under a nitrogen atmosphere while cooling on ice, and while slowly adding dropwise 6 ml of methacrylic acid chloride. After completion of the dropwise addition, the mixture was further stirred at room temperature for 48 hours. Upon completion of the reaction, 500 ml of water was added to the reaction solvent, extraction was performed with ethyl acetate, and the extract was washed. Anhydrous magnesium sulfate was added to the obtained solution for drying. The desiccant was filtered out, the solvent was distilled off under reduced pressure, and then the crude product was dissolved in 100 ml of acetic acid, heated at 100°C for 1 hour, and stirred. Upon completion of the reaction, 500 ml of water was added and the precipitate was filtered out and washed with water. This was dried under reduced pressure and then recrystallized from ethanol to obtain 5.0 g (13 mmol) of 4-(6-methacryloyloxyhexyloxy)biphenyl-4'-carboxylic acid as a white powder. (Yield: 50%)
· Measured values: ¹H-NMR (δ, DMSO-d₆): 1.40-1.87 (8H, m), 1.95 (3H, s), 4.02 (2H, t), 4.11 (2H, t), 5.66 (1H, s), 6.02 (1H, s), 7.04 (2H, d), 7.68 (2H, d), 7.75 (2H, d), 8.12 (2H, d), 10.6 (1H, s)
. Calculated: C₂₃R₂₆O₅ (382): C, 72.23%; H, 6.85%; O, 20.92%
. Found: C, 72.30%; H, 6.90%; O, 20.80%

After substitution with nitrogen, 8.0 g (50 mmol) of phosphoryl chloride was slowly added dropwise to 10 ml of DMF while cooling at 0°C, and the mixture was stirred at room temperature for 1 hour. This was then cooled to 0°C, and 5.4 g (30 mmol) ofN-methylcarbazole was dissolved in 13 ml of 1,2-dichloroethane and added dropwise thereto. The mixture was then heated to 90°C over a period of 1 hour, and heated stirring was carried out for 8 hours. Upon completion of the reaction, 500 ml of water was added, the organic layer was extracted with dichloromethane, and the extracted organic layer was dried over anhydrous magnesium sulfate. After filtering out the anhydrous magnesium sulfate, the solvent was distilled off under reduced pressure and the obtained crude product was purified by silica gel chromatography using a developing solvent (dichloromethane:hexane = 3:1 (volume ratio)), to obtain 4.8 g (23 mmol) of 3-formyl-9-methylcarbazole crystals. (Yield: 77%)
· Measured values: ¹H-NR4R (δ, CDCl₃): 3.89 (3H, s), 7.30-8.1 (4H, m), 8.05 (1H, d), 8.15 (1H, d), 8.60 (1H, s), 10.10 (1H, s)
· Calculated: C₁₄H₁₁NO: C, 80.36%; H, 5.30%; N, 6.69%, 0,7.65%.
· Found: C, 80.3%; H, 5.36%; N, 6.74%; O, 7.60%.

After dissolving 2.9 g (14 mmol) of 3-formyl-9-methylcarbazole, 1.2 g (17 mmol) of hydroxylamine hydrochloride, 3.0 g (50 mmol) of acetic acid and 2.0 g (25 mmol) of pyridine in 10 ml of DMF, the mixture was heated and stirred at 140°C for 5 hours. Upon completion of the reaction, 500 ml of water was added, and dichloromethane and hydrochloric acid were used for extraction and washing, in that order. After drying the obtained organic layer over anhydrous magnesium sulfate, the anhydrous magnesium sulfate was filtered out and the solvent was distilled off under reduced pressure. The obtained crude product was purified by silica gel chromatography using a developing solvent (dichloromethane:hexane = 3:1 (volume ratio)). A hexane/ethanol mixed solvent was used for recrystallization to obtain 2.1 g (10 mmol) of 3-cyano-9-methylcarbazole crystals. (Yield: 71%)
· Measured values: ¹H-NMR (δ, CDCl₃): 3.89 (3H, s), 7.25-8.1 (5H, m), 8.15 (1H, d), 8.60 (1H, s)
· Calculated: C₁₄H₁₀N₂ (206): C, 81.53%; H, 4.89%; N, 13.58%.
· Found: C, 81.50%; H, 5.00%; N, 13.50%.

After dissolving 3-cyano-9-methylcarbazole (3.1 g, 15 mmol), sodium azide (15 g, 230 mmol) and ammonium chloride (12 g, 230 mmol) in 110 ml of dehydrated DMF, the mixture was heated and stirred at 140°C for 10 hours. It was then cooled to room temperature, and the reaction mixture was poured into 500 ml of water, producing a precipitate. The precipitate was filtered out and washed with water. The obtained crude product was recrystallized from methanol to obtain 3.2 g (13 mmol) of 3-(5-tetrazolyl)-9-methylcarbazole crystals. (Yield: 87%)
· Measured values: ¹H-NMR (δ, DMSO-d₆): 3.89 (3H, s), 7.30-8.1 (4H, m), 8.15 (1H, d), 8.60 (1H, s).
· Calculated: C₁₄H₁₁N₅ (249): C, 67.46%; H, 4.45%; N, 28.09%.
· Found: C, 67.52%; H, 4.40%; N, 28.08%.

After adding 20 ml of thionyl chloride to 2.5 g (6.6 mmol) of 4-(6-methacryloyloxyhexyloxy)biphenyl-4'-carboxylic acid, the mixture was heated and stirred at 60°C for 2 hours. The excess thionyl chloride was then distilled off with an aspirator. Next, 20 ml of dehydrated pyridine and 2.5 g (10 mmol) of 3-(5-tetrazolyl)-9-methylcarbazole were added and the mixture was heated and stirred at 140°C for 24 hours.
This was cooled to room temperature, and then a dilute hydrochloric acid aqueous solution was added and the precipitated solid was recovered. The solid was dissolved in methylene chloride and washed 3 times with water, and then the obtained organic layer was dried over anhydrous magnesium sulfate. After filtering off the anhydrous magnesium sulfate, the solvent was distilled off under reduced pressure. The obtained crude product was purified by silica gel chromatography using a developing solvent (chloroform:tetrahydrofuran = 1:1). Further recrystallization using an ethanol/dichloromethane mixed solvent yielded 2.5 g (4.2 mmol) of 2-{4'-(6-methacryloyloxyhexyloxy)biphenyl-4-yl}-5-(9-methylcarbazol -3-yl)-1,3,4-oxadiazole (monomer 1) as pale yellow crystals. (Yield: 64%)
· Measured values: ¹H-NMR (δ, CDCl₃): 1.45-1.89 (8H, m), 1.96 (3H, s), 4.03 (2H, t), 4.18 (2H, t), 5.55 (1H, s), 6.11 (1H, s), 6.98 (2H, d), 7.30-7.75 (8H, m), 8.21 (4H, m), 8.91 (1H, s)
· Calculated: C₃₇H₃₅N₃O₄ (585): C, 75.88%; H, 6.02%; N, 7.17%; O, 10.93%.
· Found: C, 75.86%; H, 6.00%; N, 7.15%; O, 10.99%.

· Synthesis of monomer 2: [2-{4'-(11-methacryloyloxyundecyloxy)phenyl}-5-(4-N,N-diphenylami nophenyl)-1,3,4-oxadiazole]

After dissolving 10 g (72 mmol) of 4-hydroxybenzoic acid and 10 g (72 mmol) of potassium carbonate in 100 ml of DMF, a microspatula of potassium iodide was added and the mixture was heated and stirred at 60°C. Also, a solution of 15 g (60 mmol) of 11-bromo-1-undecanol in 20 ml of DMF was added dropwise, and the mixture was heated and stirred at 90°C for 5 hours. Next, 500 ml of water was added to the reaction mixture, the pH was adjusted, and a precipitate was obtained. The precipitate was filtered to obtain a white solid. The white solid was dried under reduced pressure, and the recovered white solid was recrystallized from chloroform to obtain 12.1 g (39 mmol) of 4-(11-hydroxyundecyloxy)benzoic acid as a white powder. (Yield: 54%)
· Measured values: ¹H NMR (300 MHz, CDCl₃): 1.26-1.75 (18H, m), 3.63 (2H, t), 4.26 (2H, t), 6.83 (2H, d), 7.94 (2H, d), 10.7 (1H, s)

After adding 100 ml of 1,4-dioxane to 8.0 g (26 mmol) of 4-(11-hydroxyundecyloxy)benzoic acid and 5.5 ml of triethylamine, the mixture was stirred under a nitrogen atmosphere while cooling on ice, and while slowly adding dropwise 6 ml of methacrylic acid chloride. Upon completion of the dropwise addition, the mixture was further stirred at room temperature for 48 hours. Next, 500 ml of water was added to the reaction solvent, extraction was performed with ethyl acetate, and the obtained organic layer was washed. Anhydrous magnesium sulfate was added to the organic layer for drying. The anhydrous magnesium sulfate was filtered out and the solvent was distilled off under reduced pressure, yielding a crude product. The crude product was dissolved in 100 ml of acetic acid, and heated and stirred at 100°C for 1 hour. Upon completion of the reaction, 500 ml of water was added and a precipitate was produced. The precipitate was filtered out and washed with water. This was dried under reduced pressure and then recrystallized from ethanol to obtain 6.0 g (16 mmol) of 4-(11-methacryloyloxyundecyloxy)benzoic acid as a white powder.
(Yield: 62%)
· Measured values: ¹H-NMR (δ, DMSO-d₆): 1.40-1.87 (18H, m), 1.95 (3H, s), 4.02 (2H, t), 4.11 (2H, t), 5.66 (1H, s), 6.02 (1H, s), 7.04 (2H, d), 8.12 (2H, d), 10.6 (1H, s)

After dissolving N,N-diphenyl-p-cyanoaniline (4.1 g, 15 mmol), sodium azide (15 g, 230 mmol) and ammonium chloride (12 g, 230 mmol) in 110 ml of dehydrated DMF, the mixture was heated and stirred at 140°C for 10 hours. It was then cooled to room temperature, and the obtained reaction mixture was poured into 500 ml of water, producing a precipitate. The precipitate was filtered out and washed with water. The obtained crude product was recrystallized from methanol to obtain 4.1 g (13 mmol) of N,N-diphenyl-p-(5-tetrazolyl)aniline crystals. (Yield: 87%)
· Measured values: ¹H-NMR (300 MHz, DMSO-d₆): 6.60 (4H, d), 6.69 (2H, d), 6.89 (2H, t), 7.25-7.30 (4H, m), 8.10 (2H, d), 8.60 (1H, s)

After adding 20 ml of thionyl chloride to 2.5 g (6.6 mmol) of 4-(11-methacryloyloxy)undecyloxy}benzoic acid, the mixture was heated and stirred at 60°C for 2 hours. The excess thionyl chloride was then distilled off with an aspirator. Next, 20 ml of dehydrated pyridine and 3.1 g (10 mmol) of N,N-diphenyl-p-(5-tetrazolyl)aniline were added, and the mixture was heated and stirred at 140°C for 24 hours. After cooling the obtained reaction mixture to room temperature, a dilute hydrochloric acid aqueous solution was added and the precipitated solid was recovered. The solid was dissolved in methylene chloride and washed 3 times with water, and then dried over anhydrous magnesium sulfate. After filtering off the anhydrous magnesium sulfate, the solvent was distilled off under reduced pressure. The obtained crude product was purified by silica gel chromatography using a developing solvent (chloroform:tetrahydrofuran = 1:1). It was further recrystallized using an ethanol/dichloromethane mixed solvent, to obtain 2.7 g (4.2 mmol of 2-{4-(11-methacryloyloxyundecyloxy)phenyl}-5-{4-(N,N-diphenylamino)phenyl}-1,3,4-oxadiazole (monomer 2) as pale yellow crystals. (Yield: 64%)
· Measured values: ¹H NMR (300 MHz, CDCl₃): 1,45-1.89 (18H, m), 1.96 (3H, s), 4.03 (2H, t), 4.18 (2H, t), 5.55 (1H, s), 6.11 (1H, s), 6.98 (2H, d), 7.10-7.55 (8H, m), 7.56 (2H, d), 7.72 (2H, d), 7.95 (2H, d), 8.21 (2H, d).
· Calculated: C₄₁H₄₅N₃O₄: C, 76.49; H, 7.05; N, 6.53; O, 9.94
· Found: C, 76.80; H, 7.23; N, 6.33; O, 9.64

The 2 different monomers obtained as described above (molar ratio of monomer 1:monomer 2 = 0.7:0.3, total: 1.0 g) and 2,2'-azobisisobutyronitrile (1 mol% with respect to the total of the 2 different monomers) were dissolved in distilled THF (5 mL), and subjected to deaeration by a freezing-pumping-thawing cycle conducted 3 or more times. The obtained mixture was heated and stirred in a sealed tube at 60°C for 48 hours. After cooling the obtained solution, it was added dropwise to a mixed solvent of methanol/toluene (20/1) (volume ratio) while stirring, upon which a precipitate was produced. The precipitate was dissolved in dichloromethane, and purified by dropwise addition into methanol/toluene (20/1) (volume ratio) for reprecipitation, repeated several times, and then vacuum dried to obtain 0.6 g of a high molecular compound (H-1) (yield: 60%). The number-average molecular weight Mn of the high molecular compound (H-1) in terms of polystyrene was 1.2 × 10⁵, and the weight-average molecular weight Mw in terms of polystyrene was 2.5 × 10⁴, as measured under [Analysis conditions 1].

### <Measurement of liquid crystallinity of high molecular compound (H-1)>

The liquid crystallinity of the high molecular compound (H-1) was confirmed by polarizing microscope observation (trade name: BX50 by Olympus Corp.) and differential scanning calorimetry (SSC-trade name: SSC-5200, DSC220C, by Seiko I&E).
The high molecular compound (H-1) was sandwiched between 2 glass panels, set on a hot stage (trade name: FP-90, FP82HT by Mettler) and observed with a polarizing microscope while heating, and exhibited the characteristic optical texture of liquid crystals (Schlieren texture) near 117°C. Heating at about 180°C or higher created a dark field, and birefringence was lost. Upon differential scanning calorimetry, a point of inflection appeared due to glass transition near 117°C. A broad peak was also confirmed near 180°C.
These results indicated that the high molecular compound (H-1) exhibited liquid crystal phase between about 117°C and 180°C.

### [Example 5]

### <Fabrication of EL element (C)>

A solution of the high molecular compound (H-1) dissolved in a 1,1,2,2-tetrachloroethane solvent to a concentration of 2.0 wt%, and a solution of metal complex (MC-2) dissolved in the same solvent mentioned above to a concentration of 2.0 wt%, were then combined at a weight ratio of 95:5 to prepare a composition (hereunder referred to as "composition 3"). An EL element (hereunder referred to as "EL element (C)") was fabricated in the same manner as Example 4, except that composition 3 was used instead of composition 2. Composition 3 was spin coated to form a film, at a rotational speed of 1600 rpm. The film thickness was approximately 85 nm.
Upon application of a voltage to the obtained EL element (C), EL luminescence was obtained from the EL element (C), having a peak at 605 nm due to the metal complex (MC-2), and the maximum luminous efficiency was 0.48 cd/A. The EL luminescence was polarized luminescence in the direction parallel to the rubbing direction, and the degree of polarization was 18 at 605 nm. The degree of polarization was measured in the same manner as Example 4.

### [Example 6]

### <Fabrication of EL element (E)>

A solution of poly(ethylenedioxythiophene)/polystyrenesulfonic acid (trade name: CLEVIOS P VP AI4083 by H.C. Starck) was used for film formation by spin coating to a thickness of 65 nm on a glass panel, which had an ITO film with a thickness of 45 nm formed thereon by sputtering, and the film was dried for 10 minutes at 200°C on a hot plate.
Next, the high molecular compound (I-1) was spin coated as a 0.8 wt% xylene solution, to form a film with a thickness of approximately 20 nm. It was then heat treated for 60 minutes at 180°C on a hot plate.
A solution of the high molecular compound (H-2) described hereunder dissolved in a xylene solution to a concentration of 1.2 wt%, and a solution of metal complex (MC-1) dissolved in a xylene solvent to a concentration of 1.2 wt%, were then combined at a weight ratio of 90:10 to prepare a composition (hereunder referred to as "composition 5"). Composition 5 was spin coated to form a film, at a rotational speed of 3000 rpm. The film thickness was approximately 70 nm. This was subjected to drying for 10 minutes at 160°C under a nitrogen gas atmosphere, and then to vapor deposition with barium to approximately 4 nm and then aluminum to approximately 80 nm as a cathode, to fabricate an EL element (E). Vapor deposition of the metals was initiated after the degree of vacuum reached at least 1 × 10⁻⁴ Pa.
Upon application of a voltage to the obtained EL element (E), EL luminescence was obtained from the EL element (E), having a peak at 530 nm due to the metal complex (MC-1), and the maximum luminous efficiency was 0.43 cd/A.

### <Fabrication of EL element (F)>

A solution of the high molecular compound (H-2) described hereunder dissolved in a xylene solution to a concentration of 1.2 wt%, and a solution of metal complex (MC-2) dissolved in a xylene solvent to a concentration of 1.2 wt%, were combined at a weight ratio of 90:10 to prepare a composition (hereunder referred to as "composition 6"). EL element (F) was fabricated by the same method as for fabrication of EL element (E), except that composition 6 was used instead of composition 5. Upon application of a voltage to the obtained EL element (F), EL luminescence was obtained from the EL element (F), having a peak at 610 nm due to the metal complex (MC-2), and the maximum luminous efficiency was 0.15 cd/A.

### <Synthesis of high molecular compound (H-2)>

High molecular compound (H-2) was synthesized in the following manner.
To a 200 mL flask there were added 2.4925 g (5.00 mmol) of 1,4-bis(4,4,5,5-tetramethyl-1,3,2-dioxaborolan-2-yl)-2,5-dihexylbenzen e, 2.5781 g (4.00 mmol) of 9,9-bis(4-*n*-hexylphenyl)-2,7-dibromofluorene, 0.4592 g (1.00 mmol) of N,N-bis(4-bromophenyl)-N-(4-*sec*-butylphenyl)amine and 50 mL of toluene. The mixture was heated under an argon gas atmosphere, 1.8 mg of palladium acetate and 10.6 mg of tris(2-methoxyphenyl)phosphhine were added, and 16.6 mL of a 20 wt% tetraethylammonium hydroxide aqueous solution was added dropwise at 105°C. The mixture was stirred for 21 hours at 105°C, after starting dropwise addition of the base.
Next, 611.3 mg of phenylboric acid, 1.8 mg of palladium acetate, 10.6 mg of tris(2-methoxyphenyl)phosphine and 30 mL of toluene were further added, and the mixture was stirred for 8 hours.
After removing the aqueous layer, 3.04 g of sodium N,N-diethyldithiocarbamate trihydrate and 30 mL of ion-exchanged water were added, and the mixture was stirred at 85°C for 2.5 hours. After separating the organic layer from the aqueous layer, the organic layer was rinsed with ion-exchanged water (2 times), 3 wt% aqueous acetic acid (2 times) and ion-exchanged water (2 times) in that order. The organic layer was dropped into methanol, and the precipitated solid was deposited, filtered and then dried to obtain a solid. The solid was dissolved in toluene and the solution was passed through a silica gel- and alumina-packed column that had been previously passed through with toluene, the eluate that passed through was dropped into methanol to precipitate a polymer, and the precipitate was filtered and then dried, to obtain 3.088 g of a high molecular compound (H-2). The number-average molecular weight and weight-average molecular weight in terms of polystyrene, measured under Analysis conditions 1, were Mn = 4.5 × 10⁵ and Mw = 8.0 × 10⁵, respectively.
The high molecular compound (H-2) is a high molecular compound with the following repeating unit in the following molar ratio (calculated based on the starting materials).

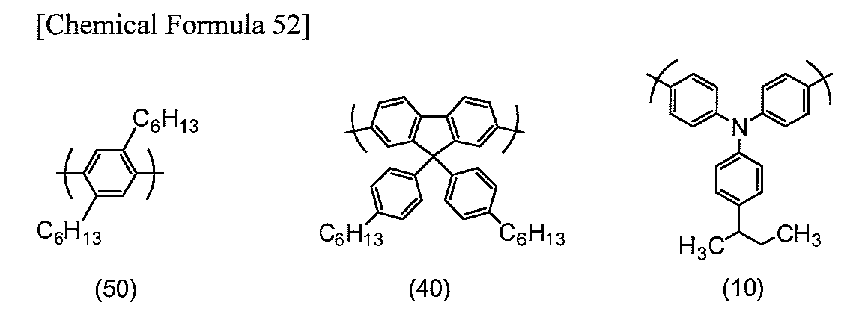

### Industrial Applicability

The metal complex of the invention and a composition comprising it are useful for production of a light emitting element, such as an organic electroluminescence element.

## Claims

1. A metal complex having a structure represented by the following formula (1): 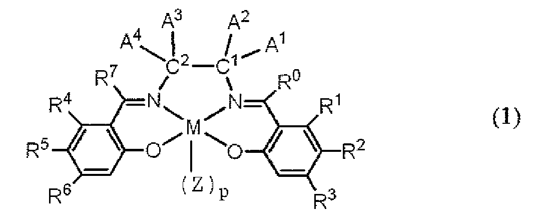 wherein M is a metal atom selected from the group consisting of copper, zinc, ruthenium, silver, osmium, rhenium, iridium, platinum, gold and lanthanum; C¹ and C² are each an sp3 carbon atom; A¹-A⁴ each independently represent hydrogen or a C3 or greater alkyl group, with at least 2 of them representing C3 or greater alkyl groups; this is with the proviso that A¹ and A³ may bond together to form a C4 or greater alkylene group and form a ring together with C¹ and C²; the alkylene group may be optionally substituted; R⁰-R⁷ each independently represent hydrogen, a halogen atom, a C6 or greater alkyl group optionally substituted with fluorine, a C12 or greater aralkyl group optionally substituted with fluorine, a C12 or greater alkaryl group optionally substituted with fluorine, a C6 or greater alkoxy group optionally substituted with fluorine, a C12 or greater arylalkoxy group optionally substituted with fluorine or a C 12 or greater alkoxyaryl group optionally substituted with fluorine, and at least one of R⁰-R⁷ is a C6 or greater alkyl group optionally substituted with fluorine, a C12 or greater aralkyl group optionally substituted with fluorine, a C12 or greater alkaryl group optionally substituted with fluorine, a C6 or greater alkoxy group optionally substituted with fluorine, a C12 or greater arylalkoxy group optionally substituted with fluorine or a C12 or greater alkoxyaryl group optionally substituted with fluorine; Z represents a monovalent monodentate ligand, and p is the number of monodentate ligands, represented by ("valency of central metal atom M" - 2).

2. The metal complex according to claim 1, having a structure represented by the following formula (2): 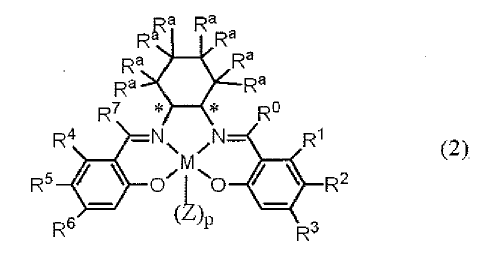 wherein R^{a} represents hydrogen, a C1 or greater alkyl group optionally substituted with fluorine, a C6 or greater aryl group, a C7 or greater aralkyl group optionally substituted with fluorine, a C7 or greater alkaryl group optionally substituted with fluorine, a C1 or greater alkoxy group optionally substituted with fluorine, a C7 or greater arylalkoxy group optionally substituted with fluorine or a C7 or greater alkoxyaryl group optionally substituted with fluorine; multiple R^{a} groups may be the same or different; R⁰-R⁷, Z and p have the same definitions as in formula (1), and "*" represents a chiral carbon atom.

3. A metal complex according to claim 2, wherein at least one R^{a} is a C3 or greater alkyl group optionally substituted with fluorine, a C7 or greater aralkyl group optionally substituted with fluorine, a C7 or greater alkoxyaryl group optionally substituted with fluorine, a C3 or greater alkoxy group optionally substituted with fluorine, a C7 or greater arylalkoxy group optionally substituted with fluorine or a C7 or greater alkoxyaryl group optionally substituted with fluorine.

4. The metal complex according to any one of claims 1 to 3, wherein M is a metal atom selected from the group consisting of ruthenium, silver, osmium, rhenium, platinum, iridium, gold and lanthanum.

5. The metal complex according to any one of claims 1 to 4, which exhibits a liquid crystal phase.

6. A composition comprising the metal complex according to any one of claims 1 to 5, and a charge transporting organic compound.

7. The composition according to claim 6, wherein the charge transporting organic compound exhibits a liquid crystal phase.

8. The composition according to claim 6 or 7, which further comprises a solvent or a dispersing medium.

9. A film obtained using the metal complex according to any one of claims 1 to 5 or the composition according to any one of claims 6 to 8.

10. The film according to claim 9, which is subjected to orientation treatment in at least one direction within the plane.

11. A light emitting element comprising the film according to claim 9 or 10.

12. The light emitting element according to claim 11, which generates polarized luminescence.

13. A planar light source employing the light emitting element according to claim 11 or 12.

14. A lighting fixture employing the light emitting element according to claim 11 or 12.
